Europäisches Patentamt

European Patent Office

Office européen des brevets

(19)

(11) Publication number: **0 200 425 B1**

(12) **EUROPEAN PATENT SPECIFICATION**

(45) Date of publication of patent specification: **27.12.91**  (51) Int. Cl.⁵: **C12N 15/80**, C12N 9/00, C12N 15/69

(21) Application number: **86302873.4**

(22) Date of filing: **17.04.86**

(54) Recombinant DNA expression vectors and DNA compounds.

(30) Priority: **22.04.85 US 725870**
**18.11.85 US 799384**

(43) Date of publication of application:
**10.12.86 Bulletin 86/45**

(45) Publication of the grant of the patent:
**27.12.91 Bulletin 91/52**

(84) Designated Contracting States:
**BE CH DE FR GB IT LI NL SE**

(56) References cited:
**EP-A- 0 107 823**
**EP-A- 0 135 291**
**EP-A- 0 177 243**
**EP-A- 0 215 539**

**SCIENCE, vol. 224, 11th May 1984, pages 610-612, Washington, DC, US; I.J. HOLLANDER et al.: "A pure enzyme catalyzing penicillin biosynthesis"**

**Idem**

(73) Proprietor: **ELI LILLY AND COMPANY**
**Lilly Corporate Center**
**Indianapolis Indiana 46285(US)**

(72) Inventor: **Ingolia, Thomas Dominick**
**1107 Kokomo Lane**
**Indianapolis Indiana 46241(US)**
Inventor: **Queener, Stephen Wyatt**
**4270 Melbourne East Drive**
**Indianapolis Indiana 46208(US)**
Inventor: **Samson, Suellen Mary**
**5812 San Clemente Drive**
**Indianapolis Indiana 46226(US)**
Inventor: **Skatrud, Paul Luther**
**1045 Fiesta Drive**
**Greenwood Indiana 46142(US)**

(74) Representative: **Hudson, Christopher Mark et al**
**Erl Wood Manor**
**Windlesham Surrey GU20 6PH(GB)**

EP 0 200 425 B1

BIOLOGICAL ABSTRACTS, vol. 79, no. 5, 1985, page AB-358, abstract no. 39805, Philadelphia, US; C.-P. PANG et al.: "Purification of isopenicillin N synthetase", & BIOCHEM J 222(3), 789-796, 1984

Idem

BIOLOGICAL ABSTRACTS/RRM, abstract no. 30002868, Philadelphia, US; J.E. BALDWIN et al.: "Amino-terminal amino-acid sequence and some properties of isopenicillin-N-synthetase from cephalosporium-acremonium", & FEBS LETTERS (NETHERLANDS) 1985, vol. 188, no. 2, pages 253-256

Idem

J.D. WATSTON et al.: "Recombinant DNA, a short course", 1983, page 85, Scientific American Books, New York, US

NATURE, vol. 318, 14th November 1985, pages 191-194, London, GB; S.M. SAMSON et al.: "Isolation, sequence determination and expression in Escherichia coli of the isopenicillin N synthetase gene from Cephalosporium acremonium"

JOURNAL OF THE CHEMICAL SOCIETY, CHEMICAL COMMUNICATIONS, December 1983, pages 1317-1319, London, GB; J.E. BALDWIN et al.: "Penicillin biosynthesis. Dual pathways from a modified substrate"

**Description**

The present invention relates to a DNA sequence encoding isopenicillin N synthetase activity. Isopenicillin N synthetase catalyzes the reaction in which isopenicillin N is formed from δ-(L-α-aminoadipyl)-L-cysteinyl-D-valine. This reaction is a critical step in the biosynthesis of important antibiotics such as penicillins from Penicillium chrysogenum, Cephalosporium acremonium, and Streptomyces clavuligerus; cephalosporins from C. acremonium; and 7α-methoxycephalosporins from S. clavuligerus.

The novel DNA sequence which encodes the isopenicillin N synthetase activity was isolated from Cephalosporium acremonium and has been used to construct recombinant DNA expression vectors which drive expression of the activity. Two types of these expression vectors are especially useful. The first type of vector drives high-level expression of the isopenicillin N synthetase activity in E. coli, and the second type drives expression of the activity in Cephalosporium acremonium.

The E. coli-produced isopenicillin N synthetase activity has been shown in in vitro tests to form isopenicillin N from δ-(L-α-aminoadipyl)-L-cysteinyl-D-valine. Crude cell extracts from E. coli transformed with the E. coli vectors of the present invention exhibited isopenicillin N synthetase activity without any prior activation treatment. The E. coli vectors of the present invention thus provide an efficient means for obtaining large amounts of active isopenicillin N synthetase. Isopenicillin N synthetase is useful, not only for the production of isopenicillin N, but also for the condensation of tripeptides other than δ-(L-α-aminoadipyl)-L-cysteinyl-D-valine to form novel antibiotics.

The Cephalosporium vectors of the present invention are useful for purposes of strain improvement. Cephalosporium is an economically important organism useful in the production of penicillin and cephalosporin antibiotics. Transformation of Cephalosporium with certain recombinant DNA expression vectors of the present invention will result in higher in vivo levels of isopenicillin N synthetase in the transformants, which thus exhibit increased efficiency and yield of fermentations involving these transformants.

The DNA compounds encoding isopenicillin N synthetase are readily modified to construct expression vectors which increase the efficiency and yield of fermentations involving other organisms, such as Penicillium chrysogenum and Streptomyces clavuligerus. Although the isopenicillin N synthetase-encoding DNA of the present invention was isolated from Cephalosporium acremonium, the present DNA compounds can be used to construct vectors which drive expression of isopenicillin N synthetase activity in a wide variety of host cells, as the E. coli vectors of the present invention illustrate. All organisms that produce penicillins and cephalosporins utilize the common precursors δ-(L-α-aminoadipyl)-L-cysteinyl-D-valine and isopenicillin N. Therefore, the isopenicillin N synthetase-encoding DNA compounds of the present invention can be used to produce vectors useful for improving efficiency and yield of fermentations involving penicillin and cephalosporin antibiotic-producing organisms of all genera.

The DNA compounds of the present invention are derived from genomic DNA of Cephalosporium acremonium and are significantly homologous in nucleotide sequence to the DNA compounds encoding isopenicillin N synthetase activity in Streptomyces clavuligerus, Penicillium chrysogenum, and other isopenicillin N synthetase-producing organisms. Because of this homology, the isopenicillin N synthetase-encoding DNA compounds of the present invention can be labelled and used to screen genomic libraries of organisms that produce isopenicillin N or similar compounds for the presence of isopenicillin N synthetase-type enzymes. Many organisms comprise DNA that encodes an isopenicillin N synthetase activity substantially equivalent to the activity encoded by the DNA compounds of the present invention, and the present invention comprises those equivalent DNA compounds.

The isopenicillin N synthetase-encoding DNA compounds of the present invention were derived from Cephalosporium acremonium genomic DNA and were isolated in conjunction with the transcriptional and translational activating sequence which controls the expression of the C. acremonium isopenicillin N synthetase-encoding genomic DNA. The present invention also comprises this novel transcriptional and translational activating sequence, which has been used, as disclosed herein, to drive expression of heterologous genes in C. acremonium.

The present invention also comprises the regulatory signals of the IPS gene that are located at the 3' end of the coding strand of the coding region of the IPS gene. These 3' regulatory sequences encode the transcription termination and mRNA polyadenylation and processing signals of the IPS gene. The presence of these signals in the proper position, which is at the 3' end of the coding strand of the coding region of the gene to be expressed, in an expression vector enhances expression of the desired product encoded by the vector in Cephalosporium acremonium.

The following section provides a more detailed description of the present invention. For purposes of clarity and as an aid in the understanding of the present invention, as disclosed and claimed herein, the following items are defined below.

Antibiotic - a substance produced by a microorganism which, either naturally or with limited chemical modification, will inhibit the growth of or kill another microorganism or eukaryotic cell.

Antibiotic Biosynthetic Gene - a DNA segment that encodes an enzymatic activity which is necessary for an enzymatic reaction in the process of converting primary metabolites into antibiotics.

Antibiotic-Producing Organism - any organism, including, but not limited to, Streptomyces, Bacillus, Monospora, Cephalosporium, Podospora, Penicillium, and Nocardia, which either produces an antibiotic or contains genes which, if expressed, would produce an antibiotic.

Antibiotic Resistance-Conferring Gene - a DNA segment that encodes an activity which confers resistance to an antibiotic.

ApR - the ampicillin resistance-conferring gene.

Bifunctional Cloning Shuttel Vector - a recombinant DNA cloning vector which can replicate and/or integrate into organisms of two different taxa.

Ceph DNA - DNA from Cephalosporium acremonium

Ceph ori - Cephalosporium acremonium mitochondrial DNA which provides for extrachromosomal maintenance of a recombinant DNA vector.

Cloning - the process of incorporating a segment of DNA into a recombinant DNA cloning vector.

cos - phage λ cohesive end sequences.

Cosmid - a recombinant DNA cloning vector which can replicate in a host cell in the same manner as a plasmid but which can also be packed into phage heads.

Functional Polypeptide - a recoverable bioactive entirely heterologous or homologous polypeptide or precursor, a recoverable bioactive polypeptide comprising a heterologous polypeptide and a portion or whole of a homologous polypeptide or a recoverable bioinactive fusion polypeptide comprising a heterologous polypeptide and a bioinactivating homologous poly-peptide which can be specifically cleaved.

Genomic Library - a set of recombinant DNA cloning vectors into which segments of DNA, which substantially represent the entire genome of a particular organism, have been cloned.

HmR - the hygromycin resistance-conferring gene.

Hybridization - the process of annealing two homologous single-stranded DNA molecules to form a double-stranded DNA molecule, which may or may not be completely base-paired.

IPS - isopenicillin N synthetase-encoding DNA.

IPSp - the transcriptional and translational activating sequence of the isopenicillin N synthetase (IPS) gene of Cephalosporium acremonium.

IPSt - the transcription termination and mRNA polyadenylation and processing signals of the IPS gene.

Isopenicillin N Synthetase - an enzyme, also known as cyclase, which catalyzes the formation of isopenicillin N from δ-(L-α-aminoadipyl)-L-cysteinyl-D-valine.

KmR - the kanamycin resistance-conferring gene.

mel - the tyrosinase gene.

mRNA - messenger ribonucleic acid.

PGK - the transcriptional and translational activating sequence of the yeast Saccharomyces cerevisiae phosphoglycerate kinase gene.

Recombinant DNA Cloning Vector - any autonomously replicating or integrating agent, including, but not limited to, plasmids, comprising a DNA molecule to which one or more additional DNA molecules can be or have been added.

Recombinant DNA Expression Vector - any autonomously replicating or integrating agent, including, but not limited to, plasmids, comprising a transcriptional and translational activating sequence positioned to drive expression of a DNA segment which encodes a polypeptide or RNA of research or commercial interest.

Recombinant DNA Vector - any recombinant DNA cloning or expression vector.

Restriction Fragment - any linear DNA molecule generated by the action of one or more enzymes.

rRNA - ribosomal ribonucleic acid.

Sensitive Host Cell - a host cell that cannot grow in the presence of a given antibiotic without a DNA segment that confers resistance thereto.

TcR - the tetracycline resistance-conferring gene.

Transcriptional Activating Sequence - a DNA sequence that promotes transcription of DNA.

Transfectant - a recipient host cell that has undergone transformation by phage DNA.

Transformant - a recipient host cell that has undergone transformation.

Transformation - the introduction of DNA into a recipient host cell that changes the genotype and results in a change in the recipient cell.

Translational activating sequence - a DNA sequence which, when translated into mRNA, promotes translation of mRNA into protein.

trp - the transcriptional and translational activating sequence of the tryptophan operon of E. coli.

Brief Description of the Figures

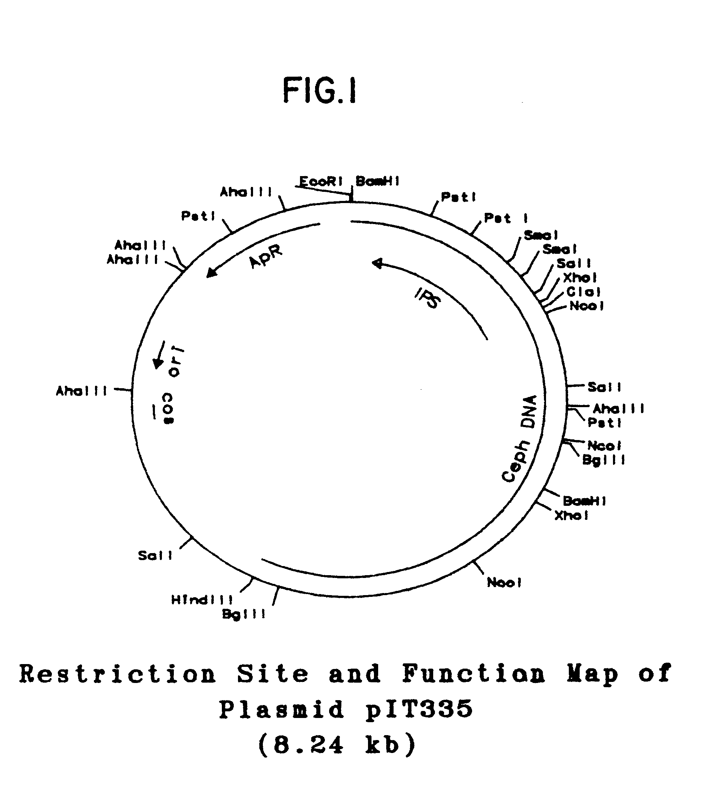

The restriction site and function maps presented in Figures 1-7 are approximate representations of the recombinant DNA vectors discussed herein. The spacing of restriction sites on the map is proportional to the actual spacing of the restriction sites on the vector, but observed restriction site distances may vary somewhat from calculated map distances. The restriction site information is not exhaustive; therefore, there may be more restriction sites of a given type on the vector than actually shown on the map.

Figure 1. A restriction site and function map of plasmid pIT335.

Figure 2. A restriction site and function map of plasmid pCZ106.

Figure 3. A restriction site and function map of plasmid pIT337.

Figure 4. A restriction site and function map of plasmid pIT221.

Figure 5. A restriction site and function map of plasmid pPS20.

Figure 6. A restriction site and function map of plasmid pPS19.

Figure 7. A restriction site and function map of plasmid pPS21.

Figure 8. A restriction site and function map of plasmid pPS21A.

Figure 9. A restriction site and function map of plasmid pPS25.

Figure 10. A restriction site and function map of plasmid pPS28.

Figure 11. A restriction site and function map of plasmid pPS29.

Figure 12. A restriction site and function map of plasmid pPS26.

Figure 13. A restriction site and function map of plasmid pPS34.

Figure 14. A restriction site and function map of plasmid pIT336.

Figure 15. A restriction site and function map of plasmid pPS35.

Figure 16. A restriction site and function map of plasmid pPS27.

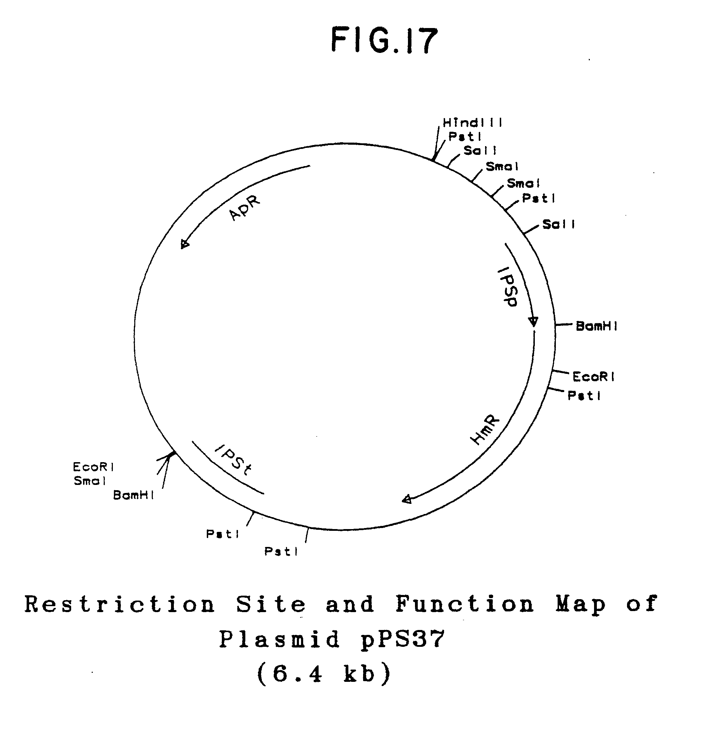

Figure 17. A restriction site and function map of pPS37.

Detailed Description of the Invention

The present invention comprises DNA compounds and recombinant DNA cloning and expression vectors which encode isopenicillin N synthetase activity. A particular DNA sequence encoding isopenicillin N synthetase activity is shown below. In the depiction, only the "sense" or coding strand of the double-stranded DNA molecule is shown, and the DNA is depicted from left to right in the 5' → 3' orientation. The nucleotide sequence is numbered; the numbers appear above the DNA sequence. Immediately below each line of DNA sequence, the amino acid residue sequence of the isopenicillin N synthetase is listed from left to right in the amino-terminus → carboxy-terminus direction. Each amino acid residue appears below the DNA which encodes it. The amino acid residue sequence is numbered; the numbers appear below the amino acid residue sequence.

# DNA Sequence Encoding Isopenicillin N Synthetase
## Activity and Corresponding Amino Acid Sequence

```
                 10              20              30              40
5'-ATG GGT TCC GTT CCA GTT CCA GTG GCC AAC GTC CCC CGA ATC GAT GTC
   MET GLY SER VAL PRO VAL PRO VAL ALA ASN VAL PRO ARG ILE ASP VAL
                  5                           10                  15

    50          60              70              80              90
TCG CCC CTA TTC GGC GAT GAC AAG GAG AAG AAG CTC GAG GTA GCT CGC
SER PRO LEU PHE GLY ASP ASP LYS GLU LYS LYS LEU GLU VAL ALA ARG
             20                      25                  30

    100             110             120             130             140
GCC ATC GAC GCC GCA TCG CGC GAC ACA GGC TTC TTT TAC GCG GTG AAC
ALA ILE ASP ALA ALA SER ARG ASP THR GLY PHE PHE TYR ALA VAL ASN
         35                      40                  45

    150             160             170             180             190
CAC GGT GTC GAC CTG CCG TGG CTC TCG CGC GAG ACG AAC AAA TTC CAC
HIS GLY VAL ASP LEU PRO TRP LEU SER ARG GLU THR ASN LYS PHE HIS
     50                      55                      60

        200             210             220             230         240
ATG AGC ATC ACG GAC GAG GAG AAG TGG CAG CTC GCC ATC CGG GCC TAC
MET SER ILE THR ASP GLU GLU LYS TRP GLN LEU ALA ILE ARG ALA TYR
65                          70              75                  80

        250             260             270             280
AAC AAG GAG CAC GAG TCC CAG ATC CGG GCG GGC TAC TAC CTG CCG ATC
ASN LYS GLU HIS GLU SER GLN ILE ARG ALA GLY TYR TYR LEU PRO ILE
             85                      90                      95

    290         300             310             320             330
CCG GGC AAG AAG GCG GTC GAA TCG TTC TGC TAC CTG AAC CCC TCC TTC
PRO GLY LYS LYS ALA VAL GLU SER PHE CYS TYR LEU ASN PRO SER PHE
             100                     105                 110
```

```
        340            350            360            370            380
AGC CCA GAC CAC CCG CGA ATC AAG GAG CCC ACC CCT ATG CAC GAG GTC
SER PRO ASP HIS PRO ARG ILE LYS GLU PRO THR PRO MET HIS GLU VAL
        115                   120                   125

        390            400            410            420            430
AAC GTC TGG CCG GAC GAG GCG AAG CAC CCG GGG TTC CGG GCC TTC GCC
ASN VAL TRP PRO ASP GLU ALA LYS HIS PRO GLY PHE ARG ALA PHE ALA
        130                   135                   140

            440            450            460            470            480
GAG AAG TAC TAC TGG GAC GTC TTC GGC CTC TCC TCC GCG GTG CTG CGC
GLU LYS TYR TYR TRP ASP VAL PHE GLY LEU SER SER ALA VAL LEU ARG
145                   150                   155                   160

            490            500            510            520
GGC TAC GCT CTC GCC CTA GGT CGC GAC GAG GAC TTC TTC ACC CGC CAC
GLY TYR ALA LEU ALA LEU GLY ARG ASP GLU ASP PHE PHE THR ARG HIS
                  165                   170                   175

    530            540            550            560            570
TCC CGC CGT GAC ACG ACG CTC TCG TCG GTC GTG CTC ATC CGT TAC CCG
SER ARG ARG ASP THR THR LEU SER SER VAL VAL LEU ILE ARG TYR PRO
                  180                   185                   190

    580            590            600            610            620
TAC CTC GAC CCG TAC CCG GAG CCG GCC ATC AAG ACG GCC GAC GAC GGC
TYR LEU ASP PRO TYR PRO GLU PRO ALA ILE LYS THR ALA ASP ASP GLY
          195                   200                   205

        630            640            650            660            670
ACC AAG CTC AGC TTC GAG TGG CAC GAG GAC GTG TCC CTC ATC ACG GTG
THR LYS LEU SER PHE GLU TRP HIS GLU ASP VAL SER LEU ILE THR VAL
        210                   215                   220

            680            690            700            710            720
TTG TAC CAG TCC GAC GTG CAG AAT CTG CAG GTC AAG ACC CCG CAG GGC
LEU TYR GLN SER ASP VAL GLN ASN LEU GLN VAL LYS THR PRO GLN GLY
225                   230                   235                   240

        730            740            750            760
TGG CAG GAC ATC CAG GCT GAC GAC ACG GGC TTC CTC ATC AAC TGC GGC
TRP GLN ASP ILE GLN ALA ASP ASP THR GLY PHE LEU ILE ASN CYS GLY
                  245                   250                   255

    770            780            790            800            810
AGC TAC ATG GCC CAT ATC ACC GAC GAC TAC TAC CCG GCC CCG ATC CAC
SER TYR MET ALA HIS ILE THR ASP ASP TYR TYR PRO ALA PRO ILE HIS
                  260                   265                   270
```

```
        820             830             840             850             860
CGC GTC AAA TGG GTC AAC GAG GAG CGC CAG TCA CTG CCC TTC TTC GTC
ARG VAL LYS TRP VAL ASN GLU GLU ARG GLN SER LEU PRO PHE PHE VAL
        275                             280                     285


        870             880             890             900             910
AAC CTG GGC TGG GAG GAC ACC ATC CAG CCG TGG GAC CCC GCG ACC GCC
ASN LEU GLY TRP GLU ASP THR ILE GLN PRO TRP ASP PRO ALA THR ALA
        290                             295                     300


        920             930             940             950             960
AAG GAT GGG GCC AAG GAT GCC GCC AAG GAC AAG CCG GCC ATC TCC TAC
LYS ASP GLY ALA LYS ASP ALA ALA LYS ASP LYS PRO ALA ILE SER TYR
305                     310                     315                     320


        970             980             990             1000
GGA GAG TAT CTG CAG GGG GGA CTG CGG GGC TTG ATC AAC AAG AAT GGT
GLY GLU TYR LEU GLN GLY GLY LEU ARG GLY LEU ILE ASN LYS ASN GLY
                325                     330                     335


1010
CAG ACC TAA-3'
GLN THR
```

wherein, A is deoxyadenyl, G is deoxyguanyl, C is deoxycytidyl, T is thymidyl, ALA is an alanine residue, ARG is an arginine residue, ASN is an asparagine residue, ASP is an aspartic acid residue, CYS is a cysteine residue, GLN is a glutamine residue, GLU is a glutamic acid residue, GLY is a glycine residue, HIS is a histidine residue, ILE is an isoleucine residue, LEU is a leucine residue, LYS is a lysine residue, MET is a methionine residue, PHE is a phenylalanine residue, PRO is a proline residue, SER is a serine residue, THR is a threonine residue, TRP is a tryptophan residue, TYR is a tyrosine residue, and VAL is a valine residue.

The DNA sequence shown above is ~63% in G and C content and encodes a polypeptide, isopenicillin N synthetase, with a calculated molecular weight of 38,476 daltons and an observed molecular weight of about 40,000 daltons.

Those skilled in the art will recognize that the DNA sequence depicted above is an important part of the present invention. The above sequence can be conventionally synthesized by the modified phosphotriester method using fully protected deoxyribonucleotide building blocks. Such synthetic methods are well known in the art and can be carried out in substantial accordance with the procedure of Itakura et al., 1977, Science 198:1056 and Crea et al., 1978, Proc. Nat. Acad. Sci. USA 75:5765. In addition, an especially preferred method is disclosed in Hsiung et al., 1983, Nucleic Acid Research 11:3227 and Narang et al., 1980, Methods in Enzymology 68:90. In addition to the manual procedures referenced above, the DNA sequence can be synthesized using automated DNA synthesizers, such as the Systec 1450A or ABS 380A DNA Synthesizers.

Due to the degenerate nature of the genetic code, which results from there being more than one codon for most of the amino acid residues and stop signal, the amino acid residue sequence of isopenicillin N synthetase depicted above can be encoded by a multitude of different DNA sequences. Because these alternate DNA sequences would encode the same amino acid residue sequence of the present invention, the present invention further comprises these alternate sequences.

In addition, there could be genetic variants of the isopenicillin N synthetase-encoding DNA of the present invention. These genetic variants would share substantial DNA and amino acid residue sequence homology with the compounds of the present invention and would have similar, if not identical, activity, but would differ somewhat from the actual compounds of the present invention. These genetic variants are also equivalent to the compounds of the present invention.

The isopenicillin N synthetase activity-encoding DNA compounds of the present invention were isolated from a strain of Cephalosporium acremonium commonly known as the Brotzu strain which is available from the American Type Culture Collection, Rockville, Maryland, under the accession number ATCC 11550. A genomic library of the total genomic DNA of the C. acremonium strain was constructed, and the genomic library was examined for the presence of sequences homologous to a set of 64 different deoxyribooligonucleotides. This set of 64 different deoxyribooligonucleotides was constructed in accordance with information obtained about the amino-terminal amino acid sequence of the C. acremonium isopenicillin N synthetase and with knowledge of the genetic code. A variety of the vectors of the genomic library were identified that were homologous to one or more of the 64 different deoxyribooligonucleotides. DNA sequencing revealed which vectors encoded the C. acremonium isopenicillin N synthetase.

After the vectors that encoded isopenicillin N synthetase were identified, one particular isopenicillin N synthetase-encoding vector was modified so as to delete most of the Cephalosporium acremonium DNA present on the vector that did not encode the isopenicillin N synthetase enzyme. The resulting vector, designated plasmid pIT335, has been transformed into E. coli K12 JA221 host cells, and the E. coli K12 JA221/pIT335 transformants have been deposited and made part of the stock culture collection of the Northern Regional Research Laboratories, Peoria, Illinois, under the accession number NRRL B-15960. A restriction site and function map of plasmid pIT335 is presented in Figure 1 of the accompanying drawings.

Plasmid pIT335 can be isolated from E. coli K12 JA221 by the procedure described in Example 1. Plasmid pIT335 was used as starting material in the construction of a plasmid, designated pIT337, that drives high-level expression of isopenicillin N synthetase in E. coli. Plasmid pIT337 was constructed by ligating the ∿1.5 kb NcoI-BamHI restriction fragment of plasmid pIT335 to the ∿8.7 kb NcoI-NcoI and ∿1.6 kb NcoI-BamHI restriction fragments of plasmid pCZ106.

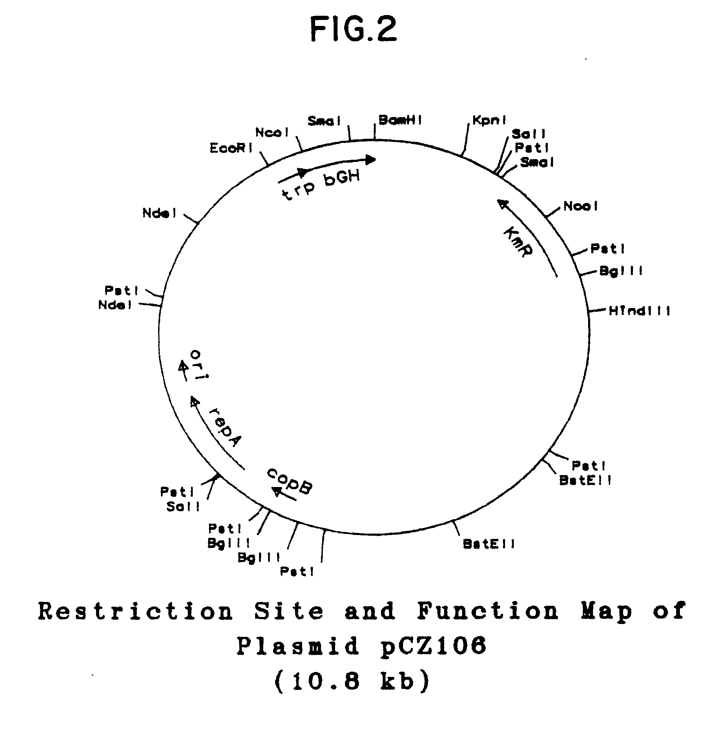

Plasmid pCZ106 comprises a runaway replicon, the trp transcriptional and translational activating sequence and operator, and a DNA sequence encoding a bovine growth hormone derivative. The use of the type of runaway replicon present on plasmid pCZ106 is described and disclosed in U.S. Patent Nos. 4,487,835; 4,499,189, and 4,495,287. Essentially, at low temperatures of about 25°C, a plasmid comprising a runaway replicon has a copy number of about ∿10-15 copies per E. coli host cell, but when the temperature is raised to about 37°C, the copy number increases to about 1,000 copies per E. coli host cell. E. coli K12 RV308/pCZ106 host cells, from which plasmid pCZ106 can be isolated, have been deposited and made part of the stock culture collection of the Northern Regional Research Laboratories, Peoria, Illinois, under the accession number NRRL B-15959. A restriction site and function map of plasmid pCZ106 is presented in Figure 2 of the accompanying drawings.

Plasmid pIT337 comprises the runaway replicon and trp transcriptional and translational activating sequence of plasmid pCZ106 and the protein-coding sequence of the isopenicillin N synthetase gene from plasmid pIT335. The ∿1.5 kb NcoI-BamHI restriction fragment of plasmid pIT335 comprises the entire proteincoding sequence for isopenicillin N synthetase, and the NcoI restriction enzyme recognition sequence, which is

$$5'-CCATGG-3'$$
$$|\;|\;|\;|\;|\;|$$
$$3'-GGTACC-5',$$

comprises the

$$5-ATG-3'$$
$$|\;|\;|$$
$$3-TAC-5'$$

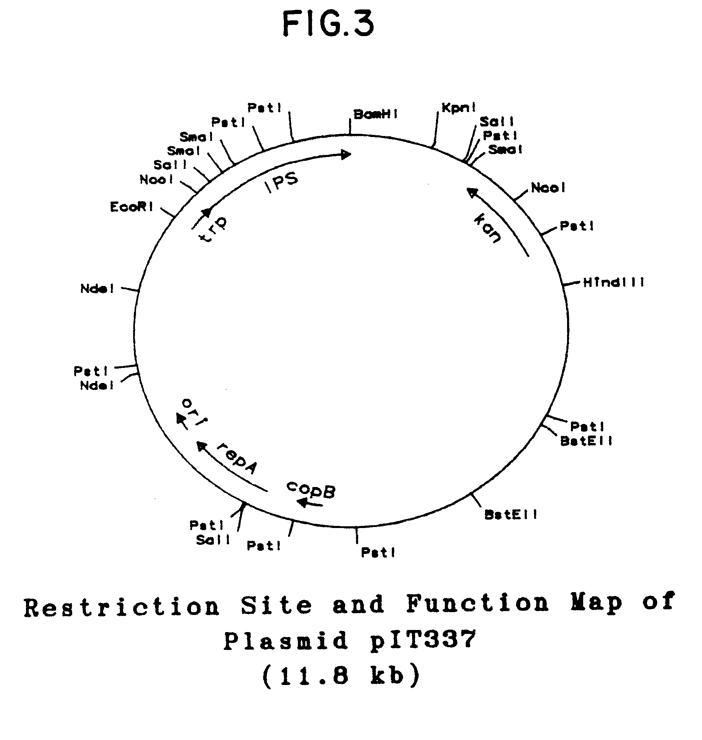

which encodes the amino-terminal methionyl residue of isopenicillin N synthetase. Plasmid pIT337 was constructed so that the trp transcriptional and translational activating sequence would be positioned to drive expression of the isopenicillin N synthetase-encoding DNA. A restriction site and function map of plasmid pIT337 is presented in Figure 3 of the accompanying drawings. Example 2 describes the construction of plasmid pIT337 in more detail.

At temperatures of about 37°C, E. coli K12 RV308 (NRRL B-15624) cells harboring plasmid pIT337 express isopenicillin N synthetase at high levels, approaching ∿9% of the total cell protein. Crude cell extracts from these E. coli K12 RV308/pIT337 transformants are able to catalyze the conversion of δ-(L-α-aminoadipyl)-L-cysteinyl-D-valine into isopenicillin N, whereas cell extracts from non-transformed E. coli K12 RV308 cells cannot catalyze this conversion. The method of assay and results of the assay for the conversion reaction are presented in Example 3.

Plasmid pIT337 provides an efficient means of producing large amounts of isopenicillin N synthetase in E. coli. Because E.. coli transformants of plasmid pIT337 express isopenicillin N synthetase at levels approaching 9% of total cell protein and because culturing E. coli is less complex than culturing organisms that naturally produce isopenicillin N synthetase, E. coli/pIT337 transformants can be used to produce recombinant isopenicillin N synthetase more efficiently and economically than nonrecombinant or "natural" isopenicillin N synthetase producers.

Isopenicillin N synthetase can be used to produce isopenicillin N from δ-(L-α-aminoadipyl)-L-cysteinyl-D-valine in a cell-free system as described in Example 3. Isopenicillin N is not only a useful antibiotic, but also is the starting material for the production of such important antibiotics as penicillin N, cephalexin, and other cephalosporins (see U.S. Patent No. 4,307,192). Perhaps the most important use of isopenicillin N synthetase is the use of the enzyme to condense tripeptides other than δ-(L-α-aminoadipyl)-L-cysteinyl-D-valine into novel β-lactam derivatives.

Cell-free extracts of penicillin-producing organisms can be used to synthesize unnatural (not produced in nature) β-lactams. The E. coli expression vectors of the present invention provide an inexpensive and efficient method of obtaining isopenicillin N synthetase, which can be used in vitro to condense tripeptides which do not naturally occur in nature to form novel antibiotics or antibiotic core structures.

The search for unnatural tripeptides that will serve as substrates for isopenicillin N synthetase can be complemented by a search for mutant isopenicillin N synthetases that will accept unnatural tripeptides as substrate. The present invention provides the starting material for such a search for a mutant isopenicillin N synthetase. E. coli is the best host for mutational cloning experiments, and the E. coli expression vectors of the present invention can be readily mutated by procedures well known in the art, such as, for example, treatment with radiation (X-ray or UV) or chemical mutagens (such as ethylmethanesulfonate, nitrosoguanidine, or methyl methanesulfonate) or site-specific mutagenesis, to obtain mutant enzymes that recognize unnatural tripeptides as substrate and catalyze the condensation of those unnatural tripeptides to unnatural β-lactams.

The present invention is not limited to the particular vectors exemplified herein. Instead, the present invention comprises DNA compounds that encode isopenicillin N synthetase activity. The DNA compounds of the present invention can be used to construct expression vectors that drive expression of isopenicillin N synthetase in any host cell in which the expression vector replicates or integrates and in which the transcriptional and translational activating sequence used to express the isopenicillin N synthetase activity functions.

Therefore, although the E. coli expression vectors exemplified herein utilize a runaway replicon functional in E. coli, the present invention comprises any E. coli expression plasmid or vector that drives expression of isopenicillin N synthetase in E. coli. Thus, the present invention comprises expression vectors which drive expression of isopenicillin N synthetase and utilize a replicon functional in E. coli, such as, for example, a replicon from such plasmids as pBR322, pACYC184, F, ColV-K94, R1, R6-5, or R100. Nor is the present invention solely limited to plasmid vectors, for the present invention also comprises expression vectors that express isopenicillin N synthetase activity and utilize integration or viral replication to provide for replication and maintenance in the host cell.

The present invention is not limited to a particular transcriptional and translational activating sequence to drive expression of the isopenicillin N synthetase activity encoding DNA. The present invention comprises the use of any transcriptional and translational activating sequence that functions in E. coli and is used to express isopenicillin N synthetase in E. coli. Many transcriptional and translational activating sequences functional in E. coli are known and are suitable for driving expression of isopenicillin N synthetase activity in E. coli. Such transcriptional and translational activating sequences include, but are not limited to, the lpp, lac, trp, tac, λpL, and λpR transcriptional and translational activating sequences.

In addition to the various E. coli transcriptional and translational activating sequences exemplified above, transcriptional and translational activating sequences from other organisms can be ligated to the present isopenicillin N synthetase-encoding DNA compounds to form expression vectors that drive expression of isopenicillin N synthetase activity in host cells in which the activating sequence functions. Although E. coli is the host best suited for isopenicillin N synthetase production and subsequent purification for in vitro use, vectors that drive expression of isopenicillin N synthetase activity in host cells other than E. coli are also useful, especially for purposes of increasing the β-lactam antibiotic-producing ability and efficiency of a given organism.

A variety of organisms produce β-lactam antibiotics. The following Table presents a non-comprehensive list of β-lactam antibiotic-producing organisms.

## TABLE I

### β-Lactam Antibiotic-Producing Organisms

| Organism | Antibiotic |
|---|---|
| Agrobacterium | various β-lactams |
| Cephalosporium acremonium | penicillins and cephalosporins |
| Chromobacterium | various β-lactams |
| Gluconobacter | various β-lactams |
| Nocardia lactamadurans uniformis | cephamycin C nocardicin |
| Penicillium chrysogenum | various penicillins and other β-lactams |
| Serratia | various β-lactams |
| Streptomyces antibioticus argenteolus | clavulanic acid asparenomycin A, MM 4550, and MM 13902 |
| cattleya chartreusis | thienamycin SF 1623 and cephamycin A and B |
| cinnamonensis clavuligerus | cephamycin A and B PA-32413-I, cephamycin C, A16886A, penicillins, cephalosporins, clavulanic acid, and other clavams |

| fimbriatus | cephamycin A and B |
| flavovirens | MM 4550 and MM 13902 |
| flavus | MM 4550 and MM 13902 |
| fulvoviridis | MM 4550 and MM 13902 |
| griseus | cephamycin A and B and carpetimycin A and B |
| halstedi | cephamycin A and B |
| heteromorphus | C2081X and cephamycin A and B |
| hygroscopicus | deacetoxy-cephalosporin C |
| lipmanii | cephamycin, penicillin N, 7-methoxycephalosporin C, A16884, MM4550, MM13902 |
| olivaceus | epithienamycin F, MM 4550, and MM 13902 |
| pana-yensis | C2081X and cephamycin A and B |
| pluracidomyceticus | pluracidomycin A |
| rochei | cephamycin A and B |
| sioyaensis | MM 4550 and MM 13902 |
| sp. OA-6129 | OA-6129A |
| sp. KC-6643 | carpetimycin A |
| tokunomensis | asparenomycin A |
| viridochromogenes | cephamycin A and B |
| wadayamensis | WS-3442-D |

Many of the foregoing β-lactam antibiotic-producing organisms are used in the pharmaceutical industry for purposes of antibiotic production. The antibiotic-producing ability of these organisms can be increased and made more efficient by increasing the intracellular concentration of the antibiotic biosynthetic enzymes during the fermentation. The isopenicillin N synthetase activity-encoding DNA compounds of the present invention can be used to construct expression vectors that, when transformed into the appropriate host cell, increase the intracellular concentration of isopenicillin N synthetase activity of the transformed host cell and thereby increase the antibiotic-producing ability and efficiency of that cell, provided that the host cell produces a β-lactam antibiotic via an intermediate reaction involving isopenicillin N synthetase activity.

A vector that will increase the intracellular concentration of isopenicillin N synthetase activity of a given host cell into which the vector is transformed requires the following elements: 1) an isopenicillin N synthetase activity-encoding DNA compound of the present invention; 2) a transcriptional and translational activating sequence that not only functions in the host cell to be transformed, but also is positioned in the correct orientation and position to drive expression of the isopenicillin N synthetase activity-encoding DNA; and 3) replication or integration functions that provide for maintenance of the vector in the host cell. Of course, the above-described vector could also comprise an antibiotic resistance-conferring gene or some other element that provides a means of selecting for host cells which contain the vector, but such selectable elements may neither be necessary nor desired when the vector integrates into the chromosomal DNA of the host cell.

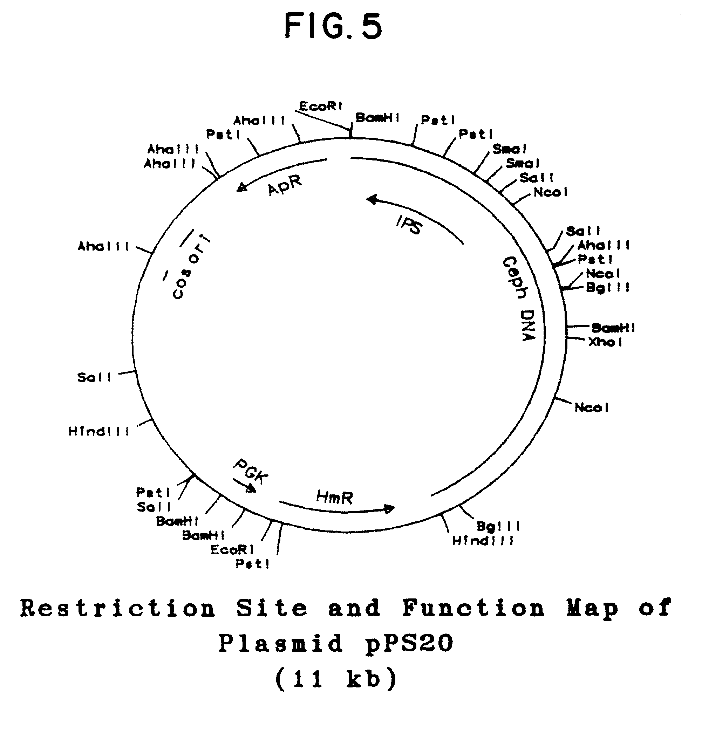

Plasmid pPS20 is an expression vector of the present invention that exemplifies the type of vector designed to increase the intracellular concentration of isopenicillin N synthetase activity in a β-lactam antibiotic-producing host cell. Plasmid pPS20 was constructed by inserting the ~2.7 kb HindIII restriction fragment of plasmid pIT221 into the single HindIII restriction enzyme recognition site of plasmid pIT335. The ~2.7 kb HindIII restriction fragment of plasmid pIT221 comprises the transcriptional and translational activating sequence of the yeast Saccharomyces cerevisiae phosphoglycerate kinase (PGK) gene ligated in the correct position and orientation to drive expression of a hygromycin resistance-conferring gene. Because the ~2.7 kb HindIII restriction fragment of plasmid pIT221 could be inserted into HindIII-digested plasmid pIT335 in either of two orientations, the ligation which produced plasmid pPS20 also produced a functionally equivalent isomer, designated plasmid pPS20.1. A restriction site and function map of plasmid pPS20 is presented in Figure 5 of the accompanying drawings; the construction of plasmids pPS20 and pPS20.1 is described in Example 4.

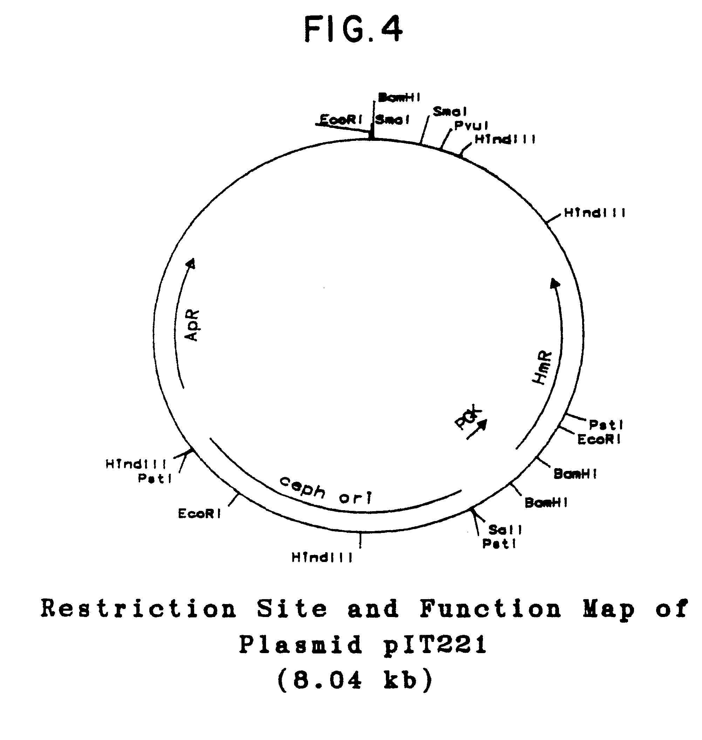

The plasmid pIT221 starting material used in the construction of plasmids pPS20 and pPS20.1 was disclosed and claimed in European Patent Application No. 85306765.0 (Publication No. 0177243) filed September 24 1985. Construction Flow Sheets I-VI and Examples 1-6 on Pages 29-57 of European Patent Application Serial No. 85306765.0 (Publication No. 0177243) describe the construction of plasmid pIT221 and are incorporated herein by reference. A restriction site and function map of plasmid pIT221 is presented in Fig. 4 of the accompanying drawings.

The ~2.7 kb HindIII restriction fragment of plasmid pIT221 comprises a hygromycin resistance-conferring gene ligated to the yeast PGK transcriptional and translational activating sequence in the correct position and orientation for expression of the hygromycin resistance-conferring activity (HmR). As disclosed in European Patent Application No. 85306765.0 the PGK-HmR gene can be used to transform Cephalosporium acremonium and related host cells to the hygromycin-resistant phenotype.

Plasmid pPS20 comprises the PGK-HmR gene, and Cephalosporium acremonium/pPS20 transformants can be selected on the basis of the hygromycin resistance-conferring activity expressed by the transformants. Plasmid pPS20 also comprises the isopenicillin N synthetase-encoding DNA of the present invention together with the genomic DNA which flanks the isopenicillin N synthetase-encoding DNA in the Cephalosporium acremonium genome.

Because plasmid pPS20 comprises almost 3 kb of the genomic DNA which was located upstream of the isopenicillin N synthetase-encoding DNA in the Cephalosporium acremonium genome, plasmid pPS20 necessarily comprises the transcriptional and translational activating sequence of the isopenicillin N synthetase-encoding DNA. Most transcriptional and translational activating sequences are encoded upstream of the DNA to be activated, although some ribosomal RNA-encoding DNA sequences are activated by transcriptional activating sequences which are not located upstream of the coding region. "Upstream" is a word used in the art of molecular biology and, in the present context, refers to DNA in the 5' direction from the 5' end of the coding strand of the isopenicillin N synthetase-encoding DNA.

The Cephalosporium acremonium transcriptional and translational activating sequence encoded on plasmid pPS20 is correctly positioned to drive expression of the isopenicillin N synthetase activity-encoding DNA, because in the construction of plasmid pPS20 no deletions or insertions affecting the transcriptional and translational activating sequence were introduced in the DNA flanking the 5' end of the coding strand of the isopenicillin N synthetase activity-encoding DNA. Plasmid pPS20 can therefore be used to increase the antibiotic-producing ability and efficiency of Cephalosporium acremonium and related host cells in which the C. acremonium transcriptional and translational activating sequence functions. This increased antibiotic-producing ability and efficiency is due to increased levels of isopenicillin N synthetase activity in the transformant, due to the presence of additional, expressed copies of the isopenicillin N synthetase activity-encoding DNA. Plasmid pPS20 also comprises a hygromycin resistance-conferring gene which functions in C. acremonium and allows for selection of C. acremonium/pPS20 transformants.

Once the Cephalosporium acremonium/pPS20 transformants are selected, however, there is no need to maintain the pressure of selection, hygromycin B, in the growth medium of the transformants. There is no need for selective pressure, because the C. acremonium/pPS20 transformants are very stable. This stability is believed to result from the plasmid pPS20 transforming C. acremonium via chromosomal integration. The present invention, however, is not limited to plasmids which drive expression of isopenicillin N synthetase activity in C. acremonium and transform via chromosomal integration. Extrachromosomally replicating expression vectors for C. acremonium are readily constructed in accordance with the teaching of U.S. Patent No. 4,492,758. U.S. Patent No. 4,492,758 describes mitochondrial DNA segments which can be inserted into a vector such as plasmid pPS20 to provide the necessary functions for extrachromosomal replication of the vector in C. acremonium.

As described above, plasmid pPS20 and one of the plasmids from which plasmid pPS20 was derived, pIT335, comprise a Cephalosporium acremonium transcriptional and translational activating sequence. Because the C. acremonium transcriptional and translational activating sequence located on plasmids pIT335 and pPS20 can be used to drive expression of a wide variety of DNA sequences, the activating sequence comprises an important part of the present invention. Although the sequence data on the C. acremonium transcriptional and translational activating sequence is limited, the activating sequence is known to be encoded on the ~500 bp SalI-NcoI restriction fragment located immediately upstream of and adjacent to the isopenicillin N synthetase activity-encoding DNA on plasmids pIT335 and pPS20. Any restriction fragment which comprises the aforementioned ~500 bp SalI-NcoI restriction fragment necessarily comprises the C. acremonium transcriptional and translational activating sequence.

There is sequence data on the Cephalosporium acremonium transcriptional and translational activating sequence encoded on plasmid pIT335. The sequence below is the DNA sequence that is upstream of the isopenicillin N synthetase activity-encoding DNA present on plasmid pIT335. Only a portion of the sequence of the ~500 bp SalI-NcoI restriction fragment that comprises the activating sequence is known, as is illustrated by the "XXXXXXXXXX" region depicted in the sequence. In order to further clarify how the activating sequence is oriented in plasmid pIT335, the restriction fragment is illustrated with single-stranded DNA overlaps characteristic of restriction enzyme SalI and NcoI cleavage.

<div align="center">

**Partial DNA Sequence**

**of the Cephalosporium acremonium**

**Transcriptional and Translational Activating Sequence**

**Encoded on Plasmid pIT335**

</div>

```
              ←~380 bp →
   5'-TCGAC XXXXXXXXXX CGAATACTTG AATATTCCTT GGTCGCTCTT
        |  ||||||||||  ||||||||||  ||||||||||  ||||||||||
      3'-G XXXXXXXXXX GCTTATGAAC TTATAAGGAA CCAGCGAGAA


         CTGATTTTCG AGGCTTCTCC TTCCGCCATC GTCGCCTCAC
         ||||||||||  ||||||||||  ||||||||||  ||||||||||
         GACTAAAAGC TCCGAAGAGG AAGGCGGTAG CAGCGGAGTG


         GCATATCTCG TCTTTCACAT CTTACACCAG CAGGACAAAC
         ||||||||||  ||||||||||  ||||||||||  ||||||||||
         CGTATAGAGC AGAAAGTGTA GAATGTGGTC GTCCTGTTTG


         CGTCAC-3'
         ||||||
         GCAGTGGTAC-5'
```

↑→beginning of isopenicillin N synthetase
coding region. "TAC" is complementary
to the 5'-ATG-3' which encodes the
amino-terminal methionyl residue of
isopenicillin N synthetase.

The Cephalosporium acremonium transcriptional and translational activating sequence can be used to drive expression of any DNA sequence, as plasmid pPS21 illustrates. Plasmid pPS21 is a derivative of plasmid pIT221 which results from the replacement of the PGK transcriptional and translational activating sequence used to drive expression of the hygromycin resistance-conferring gene with the C. acremonium transcriptional and translational activating sequence of the present invention. The replacement was accomplished by first removing the ~300 bp XmaI fragment of plasmid pIT221 to form plasmid pPS19. This XmaI deletion was performed to remove a BamHI restriction enzyme recognition site that would have interfered with the construction of pPS21. Plasmid pPS19 was then digested with BamHI and treated with the Klenow fragment of E. coli DNA Polymerase I. The BamHI digestion excises an ~230 bp BamHI restriction fragment comprising the PGK transcriptional and translational activating sequence; the Klenow treatment makes double-stranded DNA out of the single-stranded BamHI overlaps. The large, ~7.7 kb BamHI fragment of plasmid pPS19 was then ligated to the ~0.8 kb, Klenow-treated, NcoI restriction fragment of plasmid pIT335 that comprises the C. acremonium transcriptional and translational activating sequence.

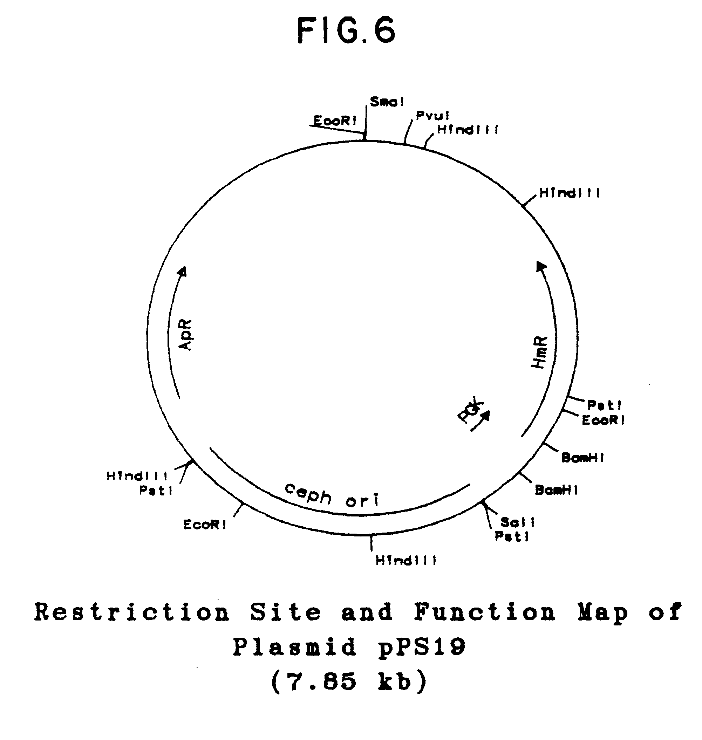

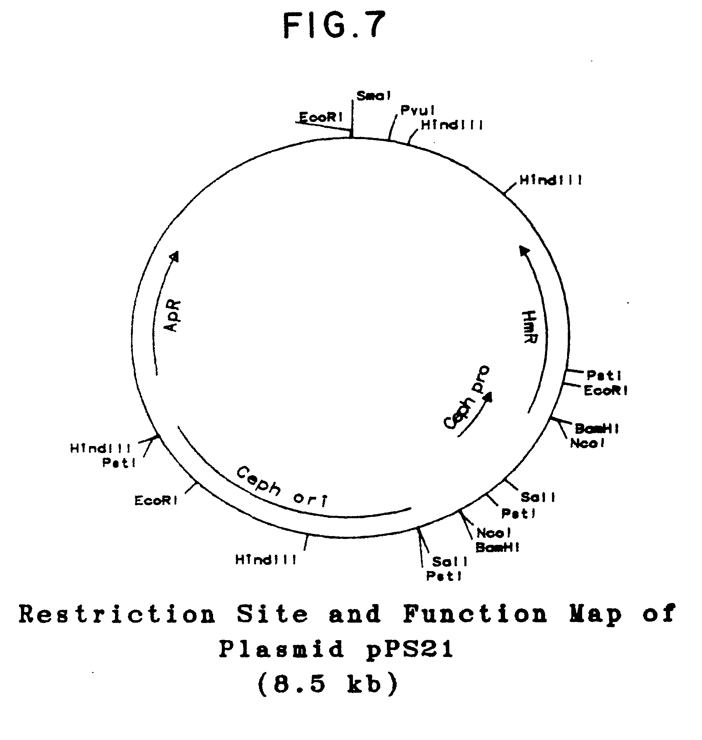

Of course, the Klenow-treated NcoI restriction fragment could insert in one of two orientations, with only one of the possible orientations achieving the desired result--the correct positioning of the Cephalosporium acremonium transcriptional and translational activating sequence to drive expression of the hygromycin resistance-conferring gene. A restriction site and function map of plasmid pPS21 is presented in Figure 7 of the accompanying drawings, and a restriction site and function map of plasmid pPS19 is presented in Figure 6 of the accompanying drawings. A more detailed description of the construction of plasmid pPS21 is presented in Example 5.

Plasmid pPS21A is another vector of the present invention that utilizes the transcriptional and translational activating sequence of the IPS gene to drive expression of a hygromycin resistance-conferring gene in Cephalosporium acremonium. A useful intermediate plasmid, designated plasmid pPS23, was used in the construction of plasmid pPS21A. Plasmid pPS23 was constructed by isolating the ∿850 bp NcoI restriction fragment of plasmid pIT335 that comprises the activating sequence of the IPS gene, attaching linkers with BamHI and NcoI-compatible, single-stranded overlaps to the ∿850 bp NcoI fragment, and ligating the resulting plasmid pIT335-derived, ∿860 bp BamHI restriction fragment to BamHI-digested plasmid pUC8. This ligation produced two plasmids, designated pPS23 and pPS23.1, that differ only with respect to the orientation of the inserted BamHI restriction fragment. Plasmid pUC8 is available from Pharmacia P-L Biochemicals, 800 Centennial Ave., Piscataway, N.J. 08854.

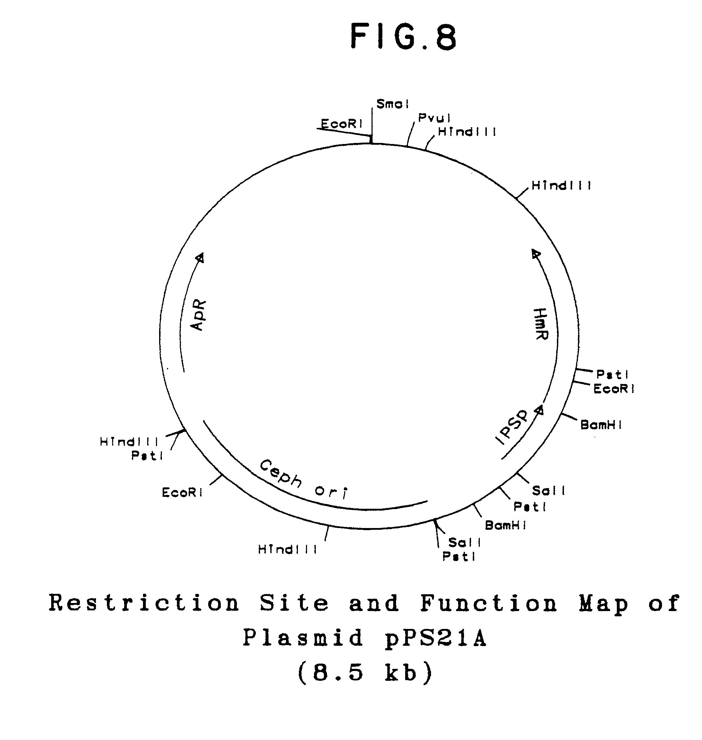

Plasmid pPS23 was digested with restriction enzyme BamHI, and the ∿860 bp BamHI restriction fragment that comprises the IPS transcriptional and translational activating sequence was isolated and ligated with BamHI-digested plasmid pPS19. This ligation produced a number of useful plasmids, including plasmid pPS21A. Plasmid pPS21A results from the ligation of the ∿0.86 kb BamHI restriction fragment of plasmid pPS23 with the ∿7.7 kb BamHI restriction fragment of plasmid pPS19 and comprises the transcriptional and translational activating sequence of the IPS gene located in the proper orientation to drive expression of the hygromycin resistance-conferring gene. The linkers used in the construction of plasmid pPS23 ensured that the proper reading frame would be maintained in plasmid pPS21A for expression of the hygromycin resistance-conferring gene. The construction of plasmid pPS21A is described in Example 7; a restriction site and function map of plasmid pPS21A is presented in figure 8 of the accompanying drawings.

Several other useful plasmids were also produced in the same ligation that produced plasmid pPS21A. Plasmid pPS22 comprises the same sequences as plasmid pPS21A, but the BamHI restriction fragment that comprises the activating sequence of the IPS gene is oriented in the opposite direction of the orientation in plasmid pPS21A. Consequently, plasmid pPS22 does not confer hygromycin resistance to Cephalosporium acremonium at high frequency, so plasmid pPS22 serves as a useful negative control in C. acremonium transformations.

Another plasmid produced in this ligation has utility both as a negative control in Cephalosporium acremonium transformations and also as a plasmid that can be used to identify C. acremonium sequences that possess transcriptional and translational activating activity. As described above, plasmid pPS19 comprises the Saccharomyces cerevisiae PGK transcriptional and translational activating sequence in the proper orientation to drive expression of the hygromycin resistance conferring gene. Digestion of plasmid pPS19 with restriction enzyme BamHI yields two fragments: one fragment is about 230 bp in size and comprises the PGK activating sequence, and the other fragment is ∿7.6 kb in size and comprises most of the coding sequence for the hygromycin resistance conferring gene.

Circularization of the ∿7.6 kb BamHI restriction fragment of plasmid pPS19 yields plasmid pPS24, which lacks a transcriptional and translational activating sequence positioned to drive expression of the hygromycin resistance-conferring gene present on the vector. Plasmid pPS24 can therefore transform Cephalosporium acremonium to hygromycin resistance by integrating into the C. acremonium DNA in such a position that an endogenous C. acremonium transcriptional and translational activating sequence drives expression of the gene. Consequently, identification of the site of integration of the plasmid pPS24 DNA in a hygromycin-resistant, C. acremonium/pPS24 transformant will also identify a C. acremonium transcriptional and translational activating sequence. Alternatively, C. acremonium DNA could be cloned into the single BamHI site on plasmid pPS24 and the resulting plasmids used to transform C. acremonium. Those plasmids that transformed C. acremonium to hygromycin resistance at a high frequency would necessarily comprise a transcriptional and translational activating sequence functional in C. acremonium.

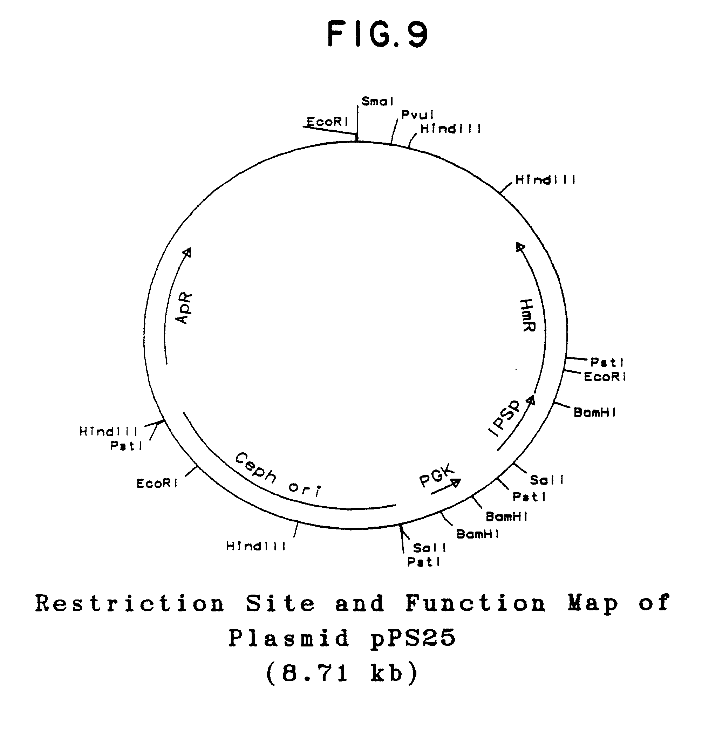

Yet other useful plasmids were produced in the same ligation that produced plasmid pPS21A. These plasmids, designated plasmids pPS25 and pPS25.1, were produced by the ligation of the two BamHI restriction fragments of plasmid pPS19 to the ∿860 bp BamHI restriction fragment of plasmid pPS23. In plasmid pPS25, both the PGK and IPS activating sequences are in the proper orientation to drive expression of the hygromycin resistance-conferring gene. Plasmid pPS25 comprises the IPS activating sequence located immediatey upstream of the coding sequence of the hygromycin resistance-conferring gene and the PGK activating sequence located immediately upstream of the IPS activating sequence. Plasmid pPS25 confers hygromycin resistance to Cephalosporium acremonium. A restriction site and function map of plasmid pPS25 is presented in Figure 9 of the accompanying drawings. The construction of plasmids pPS22, pPS24, and pPS25 is also described in Example 7.

Plasmid pPS25.1 only differs from plasmid pPS25 with respect to the orientation of the ∿0.23 kb BamHI restriction fragment that comprises the PGK activating sequence. In plasmid pPS25.1, the PGK activating sequence is not positioned in the orientation that allows the PGK activating sequence to drive expression of the hygromycin resistance-conferring gene. However, both plasmid pPS25 and pPS25.1 transform Cephalosporium acremonium to the hygromycin-resistant phenotype at the same high frequency, indicating that the PGK activating sequence is not necessary for the expression of the hygromycin-resistant phenotype.

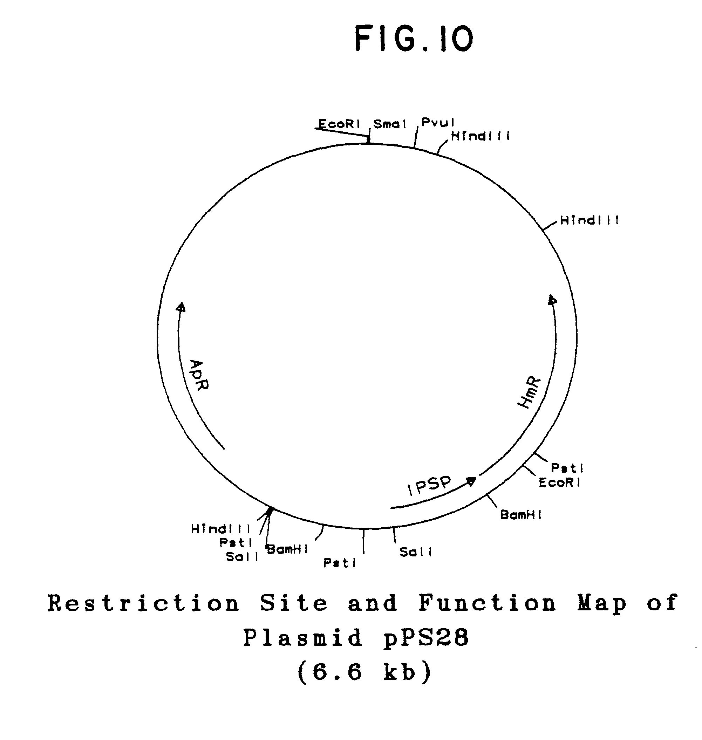

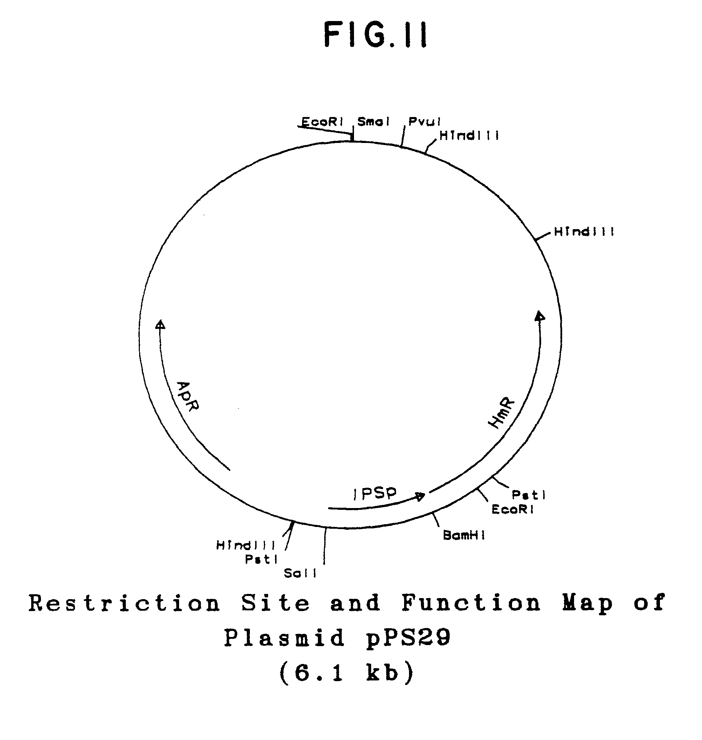

Other useful plasmids of the present invention that confer hygromycin resistance to Cephalosporium acremonium can be constructed by partial digestion of plasmid pPS21 with restriction enzyme PstI, followed by religation. Plasmid pPS28 results from deleting from plasmid pPS21A the ~1.85 kb PstI restriction fragment that comprises the Cephalosporium origin of replication. Plasmid pPS29 results from deleting from plasmid pPS21A the same PstI fragment that was deleted to get plasmid pPS28 together with the ~0.49 kb PstI restriction fragment that lies between the Cephalosporium origin of replication and the activating sequence of the IPS gene on plasmid pPS21A. Restriction site and function maps of plasmids pPS28 and pPS29 are respectively presented in Figures 10 and 11. The construction of plasmids pPS28 and pPS29 is described in Example 8.

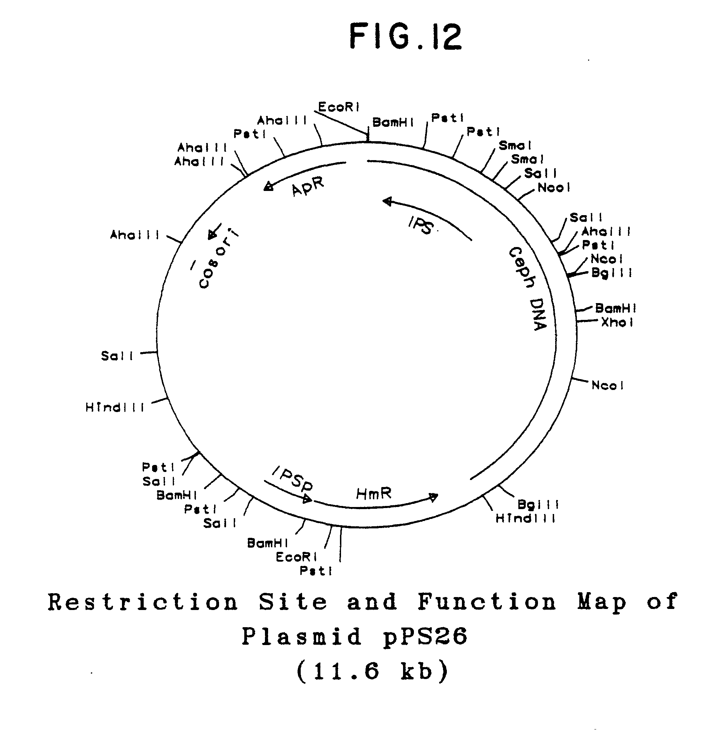

Yet another useful derivative was constructed using plasmid pPS21A as starting material. The ~3.45 kb HindIII restriction fragment of plasmid pPS21A was inserted into the single HindIII site of plasmid pIT335 to yield plasmids pPS26 and pPS26.1, which differ only with respect to the orientation of the inserted HindIII restriction fragment from plasmid pPS21A. Plasmids pPS26 and pPS26.1 comprise the intact IPS gene from Cephalosporium acremonium and the hygromycin resistance-conferring gene driven by the activating sequence of the IPS gene. The construction of plasmids pPS26 and pPS26.1 is described in Example 9, and a restriction site and function map of plasmid pPS26 is presented in Figure 12 of the accompanying drawings.

European Patent Application No. 85306765.0 (European Patent-Publication No. 0177243) describes the construction of a vector similar to plasmid pIT221, a plasmid which, as stated above, is also described and disclosed in the same application, but this similar vector further comprises Cephalosporium acremonium ribosomal RNA-encoding DNA. The plasmid, designated pPS6, has enhanced ability to integrate into C. acremonium chromosomal DNA due to the presence of the rRNA-encoding DNA. The construction of plasmid pPS6 is disclosed on pages 72 to 75, Example 13, of the above mentioned U.S. Patent Application Serial No. 654,919, and the referenced Example 13 is incorporated herein by reference. Because plasmid pPS6 comprises the same PGK-HmR gene as does plasmid pIT221, the pPS6 derivative that would result from replacement of the PGK transcriptional and translational activating sequence with the C. acremonium activating sequence of the present invention is clearly within the scope of the present invention.

Plasmid pPS6 contains an ~3.7 kb XmaI restriction fragment that comprises rRNA genes of Cephalosporium acremonium. The presence of this XmaI fragment on a plasmid increases the likelihood that the plasmid will integrate into the C. acremonium genome by homologous recombination when the plasmid is transformed into C. acremonium. Thus, plasmids pPS30 and pPS30.1 were constructed by inserting the ~3.7 kb XmaI restriction fragment of plasmid pPS6 that comprises a portion of the C. acremonium rRNA genes into the single XmaI site of plasmid pPS21A; plasmids pPS30 and pPS30.1 differ only with respect to the orientation of the XmaI restriction fragment. Plasmids pPS31 and pPS31.1 were constructed by inserting the ~3.7 kb XmaI restriction fragment of plasmid pPS6 into the single XmaI site of plasmid pPS29. The construction of plasmids pPS30, pPS30.1, pPS31, and pPS31.1 is described in Example 10.

The plasmid vectors of the present invention that utilize the transcription and translation activating sequence of the Cephalosporium acremonium IPS gene to drive expression of the hygromycin resistance-conferring gene are far superior to plasmids that utilize the Saccharomyces cerevisiae PGK activating sequence to drive expression of the hygromycin resistance-conferring gene in C. acremonium. The superiority of the present vectors is demonstrated by two observations: (1) the transformation frequency, as measured by the number of hygromycin-resistant Cephalosporium acremonium transformants per microgram of vector DNA used in the transformation, is 50 to 300 times higher when the IPS activating sequence, as opposed to the PGK activating sequence, is used to drive expression of the hygromycin resistance-conferring gene on the vector; and (2) the regeneration time, as measured by the time it takes for colonies visible to the naked eye to appear after the transformation, is about 50% less when the IPS activating sequence, as opposed to the PGK activating sequence, is used to drive expression of the hygromycin resistance-conferring gene on the vector.

The Cephalosporium acremonium transcriptional and translational activating sequence can be used to express any DNA sequence in C. acremonium, as indicated by the expression vectors described above. Thus, the present invention comprises the use of the C. acremonium transcriptional and translational activating sequence encoded within the ~0.5 kb SalI-NcoI restriction fragment of plasmid pIT335 to drive expression of any DNA sequence that encodes a useful substance.

The present invention results from the cloning of an intact, functional, Cephalosporium acremonium DNA sequence which encodes not only the amino acid sequence of isopenicillin N synthetase but also the transcriptional and translational activating sequence necessary to drive expression of isopenicillin N synthetase in C. acremonium. Likewise, the IPS gene of the present invention comprises the sequences located downstream of the coding region that are responsible for terminating transcription and for providing the mRNA polyadenylation and processing signals. Usually, the sequences responsible for transcription termination, polyadenylation, and mRNA processing are encoded within the region ~500 bp downstream of the stop codon of the coding region. Therefore, the ~0.5 kb BamHI-PstI restriction fragment that comprises the IPS carboxy-terminal-encoding DNA and downstream sequences thereof also comprises the transcription termination and mRNA polyadenylation and processing signals of the IPS gene.

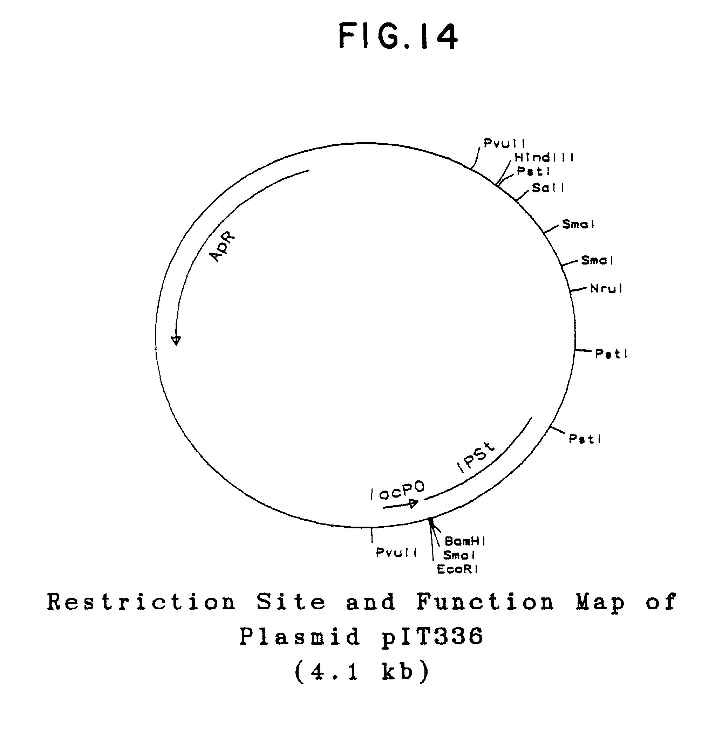

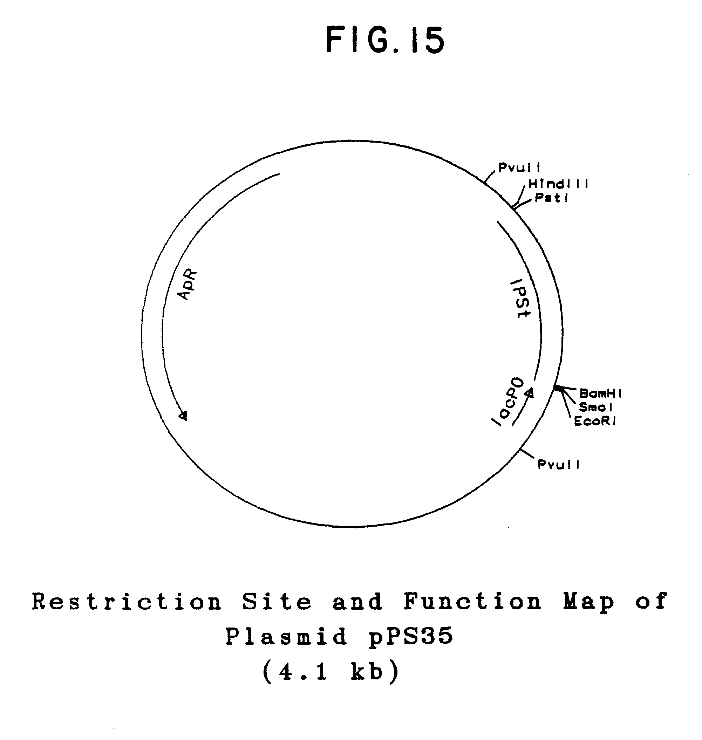

One vector, designated plasmid pPS27, has been constructed that contains the IPS transcription and translation activating sequence, followed by the hygromycin resistance-conferring gene, followed by the transcription termination and mRNA polyadenylation and processing signals of the IPS gene. To construct plasmid pPS27, the ~1.4 kb BamHI-XhoI restriction fragment of plasmid pIT335 was inserted into SalI-BamHI-digested plasmid pUC8 (SalI and XhoI overlaps are compatible) to yield plasmid pIT336 (Figure 14). Plasmid pIT336 was digested with restriction enzyme PstI and recircularized to delete all of the Cephalosporium DNA sequences from the plasmid except the ~0.5 kb BamHI-PstI restriction fragment that comprises the transcription termination and mRNA polyadenylation and processing signals of the IPS gene to yield plasmid pPS35 (Figure 15).

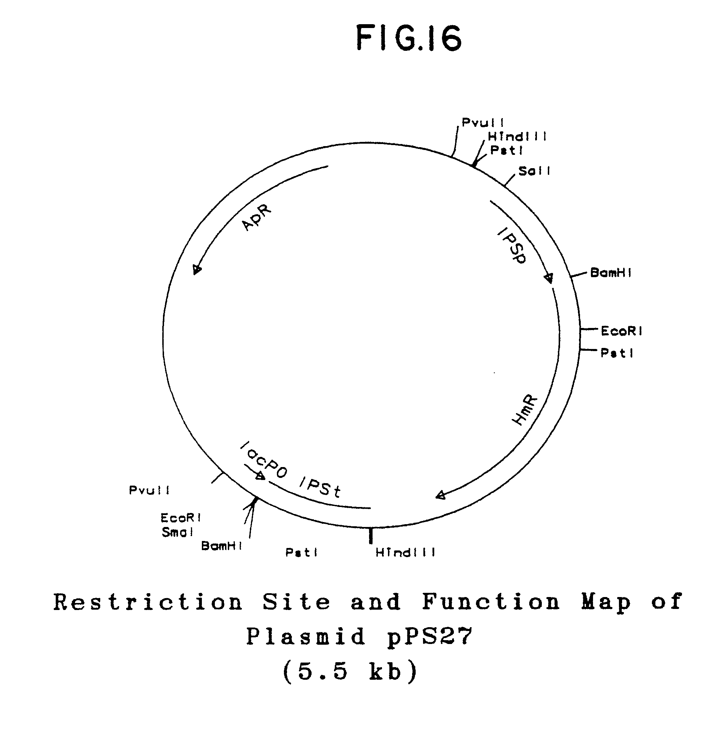

Plasmid pPS35 was then digested with restriction enzyme HindIII, and the ~2.3 kb HindIII restriction fragment of plasmid pPS29 that comprises the transcriptional and translational activating sequence of the IPS gene followed by the hygromycin resistance-conferring gene was inserted into HindIII-digested plasmid pPS35 in the proper orientation to yield plasmid pPS27. The construction of plasmid pPS27 is described in Example 11, and a restriction site and function map of plasmid pPS27 is presented in Figure 16 of the accompanying drawings.

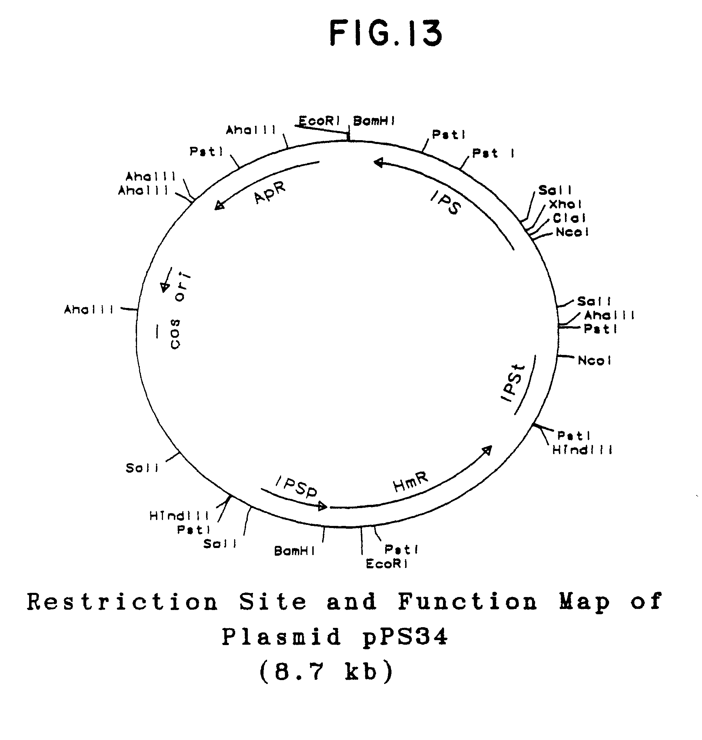

A useful derivative of plasmid pPS27 can be constructed by isolating the ~2.3 kb HindIII and ~0.5 kb HindIII-BamHI restriction fragments of plasmid pPS27 and inserting these fragments in the proper orientation into the ~5.9 kb BglII-HindIII restriction fragment of plasmid pIT335 to yield plasmid pPS34. Plasmid pPS34 comprises both the hygromycin resistance-conferring gene and also the isopenicillin N synthetase-encoding gene controlled by the regulatory elements of the IPS gene. A restriction site and function map of plasmid pPS34 is presented in Figure 13 of the accompanying drawings.

Plasmid pIT336 can be used as starting material to construct a plasmid that will integrate into the Cephalosporium acremonium genome at the locus of the isopenicillin N synthetase gene. This plasmid, designated pPS37, has utility for insertional inactivation studies, for transformation of plasmid pPS37 into a C. acremonium strain will produce IPS-deficient mutants of that strain when plasmid pPS37 integrates into the coding region of the IPS gene. Plasmid pPS37 was constructed by inserting the ~3.45 kb HindIII restriction fragment of plasmid pPS21A, which comprises the hygromycin resistance-conferring gene under the control of the activating sequence of the IPS gene, into NruI-digested plasmid pIT336. Plasmid pIT336 has a single NruI site located in the portion of the IPS coding region present on the plasmid. The insertion of the ~3.45 kb HindIII restriction fragment, which is blunt-ended by treatment with Klenow enzyme, into the NruI-digested plasmid pIT336 actually produces two plasmids, designated pPS37 and pPS37.1, which differ only with respect to the orientation of the inserted fragment. Both plasmid pPS37 and plasmid pPS37.1 are useful to transform C. acremonium to obtain hygromycin-resistant, IPS-deficient transformants.

The present invention is a pioneering invention in that it represents the first cloning and genetic engineering of a DNA sequence that encodes the enzymatic activity, often called a cyclase activity, necessary to catalyze condensation of a tripeptide substrate into a substituted $\beta$-lactam. Many organisms other than C. acremonium express a substantially similar, if not identical, cyclase activity. The similarity of cyclase activity in antibiotic-producing organisms of different genera results from a corresponding similarity of the amino acid sequence of the different cyclases and of the DNA sequence encoding the cyclase activity.

The present invention provides both an amino acid and a DNA sequence for a cyclase enzyme, specifically the isopenicillin N synthetase of Cephalosporium acremonium, and thus can be used to isolate cyclase enzyme-encoding DNA from $\beta$-lactam-producing organisms. For instance, the present DNA sequences can be used to prepare labelled probes that can, in turn, be used to find cyclase-encoding DNA sequences in the aforementioned $\beta$-lactam-producing organisms. The high G and C content of the present isopenicillin N synthetase-encoding DNA, ~63%, makes the present DNA compounds especially useful for isolating the Streptomyces clavuligerus isopenicillin N synthetase-encoding DNA. Streptomyces DNA is known to have high G and C content, often approaching 70%, so the high G and C content of the DNA of the present invention makes the present DNA compounds especially useful for isolating homologous S . clavuligerus or other Streptomyces DNA sequences. The present invention comprises DNA compounds that encode cyclase activity and further comprises expression vectors that drive expression of that cyclase activity in a variety of host organisms.

The following Examples are provided to further illustrate and exemplify the present invention but are in no way intended to limit the scope of the present invention.

Example 1

**Culture of E. coli K12 JA221/pIT335 and Isolation of Plasmid pIT335**

A. **Culture of E. coli K12 JA221/pIT335**

A lyophil of E. coli K12 JA221/pIT335 is obtained from the Northern Regional Research Laboratories, Peoria, Illinois under the accession number NRRL B-15960. The lyophil can be directly used as the "culture" in the process described below.

One liter of L-broth (10 g tryptone, 10 g NaCl, and 5 g yeast extract per liter) containing 50 $\mu$g/ml ampicillin was inoculated with a culture of E. coli K12 JA221/pIT335 and incubated in an air-shaker at 37°C until the optical density at 590 nm ($\overline{O.D._{590}}$) was ~1 absorbance unit, at which time 150 mg of chloramphenicol were added to the culture. The incubation was continued for about 16 hours; the chloramphenicol addition inhibits protein synthesis, and thus inhibits further cell division, but allows plasmid replication to continue.

B. Isolation of Plasmid pIT335

The culture prepared in Example 1A was centrifuged in a Sorvall GSA rotor (DuPont Co., Instrument Products, Biomedical Division, Newtown, CN 06470) at 6000 rpm for 5 minutes at 4°C. The resulting supernatant was discarded, and the cell pellet was washed in 40 ml of TES buffer (10 mM Tris-HCl, pH = 7.5; 10 mM NaCl; and 1 mM EDTA) and then repelleted. After discarding the supernatant again, the cell pellet was frozen in a dry ice-ethanol bath and then thawed. The thawed cell pellet was resuspended in 10 ml of a solution of 25% sucrose and 50 mM EDTA. After adding and mixing: 1 ml of a 5 mg/ml lysozyme solution; 3 ml of 0.25 M EDTA, pH = 8.0; and 100 $\mu$l of 10 mg/ml RNAse A, the solution was incubated on ice for 15 minutes. Three ml of lysing solution (prepared by mixing 3 ml 10% Triton-X 100; 75 ml 0.25 M EDTA, pH = 8.0; 15 ml of 1 M Tris-HCl, pH = 8.0; and 7 ml of water) were added to the lysozyme-treated cells, mixed, and the resulting solution incubated on ice for another 15 minutes. The lysed cells were frozen in a dry ice-ethanol bath and then thawed.

The cellular debris was removed from the solution by centrifugation at 25,000 rpm for 40 minutes in an SW27 rotor (Beckman, 7360 N. Lincoln Ave., Lincolnwood, IL 60646) and by extraction with buffered phenol. After adding 30.44 g of CsCl and ~l ml of a 5 mg/ml ethidium bromide solution, the solution volume was adjusted to 40 ml and decanted into a VTi50 ultracentrifuge tube (Beckman). After sealing the tube, the solution was centrifuged in a VTi50 rotor at 42,000 rpm for ~16 hours. The resulting plasmid band, visualized with ultraviolet light, was isolated and then placed in a Ti75 tube and rotor (Beckman) and centrifuged at 50,000 rpm for 16 hours. Any necessary volume adjustments were made using TES containing 0.761 g/ml CsCl. The plasmid band was again isolated, extracted with salt-saturated isopropanol to remove the ethidium bromide, and diluted 1:3 with TES buffer. Two volumes of ethanol were then added to the solution, followed by incubation overnight at -20°C. The plasmid DNA was pelleted by centrifuging the solution in an SS34 rotor (Sorvall) for 15 minutes at 10,000 rpm.

The ~1 mg of plasmid pIT335 DNA obtained by this procedure was suspended in 1 ml of TE buffer (10 mM Tris-HCl, pH = 8.0 and 1 mM EDTA) and stored at -20°C. A restriction site and function map of plasmid pIT335 is presented in Figure 1 of the accompanying drawings.

Example 2

Construction of Plasmid pIT337

A. **Culture of E. coli K12 RV308/pCZ106 and Isolation of Plasmid pCZ106.**

A lyophil of a culture of E. coli K12 RV308/pCZ106 is obtained from the Northern Regional Research Laboratories, Peoria, Illinois, under the accession number NRRL B-15959. The lyophil is used to inoculate 1 liter of L-broth containing 50 $\mu$g/ml kanamycin, and the inoculated broth is incubated at 25°C in an air-shaker until the O.D.$_{590}$ is between 0.5 and 1.0 absorbance units. When the culture reaches 0.5-1.0 absorbance units in optical density, the temperature is raised to 37° C and incubation is continued for 2 to 6 hours. The runaway replicon, as stated previously herein, is temperature sensitive and loses copy number control at 37° C. The 2 to 6 hour incubation at 37° C provides ample time for uncontrolled replication.

After the 2 to 6 hour incubation at 37° C, the cells are collected, and the plasmid pCZ106 DNA is isolated in substantial accordance with the procedure of Example 1B. About 5 mg of plasmid pCZ106 DNA is obtained and suspended in 5 ml of TE buffer. A restriction site and function map of plasmid pCZ106 is provided in Figure 2 of the accompanying drawings.

B. NcoI and BamHI Digestion of Plasmid pCZ106 and Isolation of the ∿8.7 kb NcoI-NcoI and ∿1.6 kb NcoI-BamHI Restriction Fragments of Plasmid pCZ106

Approximately 25 µg, corresponding to 25 µl, of the plasmid pCZ106 DNA prepared in Example 2A were added to and mixed with 10 µl of 10X BamHI reaction buffer (1.5 M NaCl; 60 mM Tris-HCl, pH = 7.9; 60 mM MgCl₂; and 1 mg/ml bovine serum albumin (BSA)), 5µl (∿50 units) restriction enzyme * BamHI, 5 µl (∿50 units) restriction enzyme NcoI, and 55 µl of H₂O. The resulting reaction was incubated at 37° C for four hours, after which time the reaction was essentially complete.

The NcoI-BamHI reaction mixture was then electrophoresed on a 1% agarose gel until the desired ∿1.6 kb NcoI-BamHI and ∿8.7 kb NcoI-NcoI fragments were clearly separated from the other digestion product, an ∿0.3 kb restriction fragment. Visualization of the electrophoresed DNA was accomplished by staining the gel in a dilute solution (0.5 µg/ml) of ethidium bromide and exposing the stained gel to long-wave UV light. After locating the desired fragments, a small slit was made in the gel in front of each of the desired fragments, and a small piece of Schleicher and Schuell (Keene, NH 03431) NA-45 DEAE membrane was placed in each slit. Upon further electrophoresis, the DNA non-covalently bound to the DEAE membrane. After the desired fragments were bound to the DEAE membrane, the membranes were removed and rinsed with low salt buffer (100 mM KCl; 0.1 mM EDTA; and 20 mM Tris-HCl, pH = 8). Next, each membrane was placed in a small tube and immersed in high salt buffer (1 M NaCl; 0.1 mM EDTA; and 20 mM Tris-HCl, pH = 8) and then incubated at 65° C for one hour to remove the DNA from the DEAE paper. After the 65° C incubation, the incubation buffer was collected and the membrane rinsed with high salt buffer. The rinse solution was pooled with the incubation buffer before collecting the desired DNA fragments.

The volume of the high salt-DNA solution was adjusted so that the NaCl concentration was 0.25 M, and then three volumes of cold, absolute ethanol were added. The resulting solutions were mixed and placed at -70° C for 10-20 minutes. After chilling, the solutions were centrifuged at 15,000 rpm for 15 minutes. After another precipitation to remove residual salt, the DNA pellets were rinsed with ethanol dried, resuspended in 20 µl of TE buffer, and constituted ∿5.0 µg each of the desired ∿1.6 kb NcoI-BamHI and ∿8.7 kb NcoI-NcoI restriction fragments of plasmid pCZ106. The purified fragments obtained were individually dissolved in 25 µl of TE buffer and stored at -20° C.

C. NcoI and BamHI Digestion of Plasmid pIT335 and Isolation of the ∿1.5 kb NcoI-BamHI Restriction Fragment that Encodes Isopenicillin N Synthetase

Approximately 25 µg, corresponding to 25 µl, of the plasmid pIT335 DNA prepared in Example 1B were digested with restriction enzymes NcoI and BamHI in substantial accordance with the procedure of Example 2B. The NcoI-BamHI-digested DNA obtained was loaded onto a 1% agarose gel and the desired ∿1.5 kb NcoI-BamHI restriction fragment was isolated in substantial accordance with the procedure of Example 2B. Approximately 5 µg of the desired fragment were obtained, suspended in 25 µl of TE buffer, and stored at -20° C.

D. Final Construction of Plasmid pIT337

Five µl of the ∿1.6 kb NcoI-BamHI and 2.5 µl of the ∿8.7 kb NcoI-NcoI restriction fragments of plasmid pCZ106 purified in Example 2B were ligated to five µl of the ∿1.5 kb NcoI-BamHI restriction fragment of plasmid pIT335 purified in Example 2C to form plasmid pIT337. The reaction volume was 30 µl and comprised the aforementioned DNA fragments, 1.1 µl (∿100 units) T4 DNA ligase, 3 µl 10X ligation buffer

*Unless otherwise noted, restriction and ligation enzymes were obtained from New England Biolabs, 32 Tozer road, beverly, MA 01915. Unit definitions herein correspond to the particular manufacturer's unit definitions.

(0.5 M Tris-HCl, pH = 7.8; 100 mM $MgCl_2$; 200 mM dithiothreitol (DTT); 10 mM ATP; and 1 mg/ml BSA), and 13.4 $\mu$l of $H_2O$. The reaction was incubated at 15°C for 2 hours, after which time the reaction was essentially complete. The ligated DNA constituted the desired plasmid pIT337 DNA. A restriction site and function map of plasmid pIT337 is presented in Figure 3 of the accompanying drawings.

Example 3

**Construction of E. coli K12 RV308/pIT337 and Assay of E. coli-Produced Isopenicillin N Synthetase**

A. **Construction of E. coli K12 RV308/pIT337**

A 50 ml culture of E. coli K12 RV308 (NRRL B-15624) in L-broth was grown to an O.D.$_{590}$ of $\sim$0.5 absorbance units. The culture was chilled on ice for ten minutes, and the cells were collected by centrifugation. The cell pellet was resuspended in 25 ml of cold 100 mM $CaCl_2$ and incubated on ice for 25 minutes. The cells were once again pelleted by centrifugation, and the pellet was resuspended in 2.5 ml of cold 100 mM $CaCl_2$ and incubated on ice overnight.

Two hundred $\mu$l of this cell suspension were mixed with the ligated DNA prepared in Example 2D and incubated on ice for 20 minutes, and then the cells were collected by centrifugation. The cell pellet was resuspended in $\sim$1 ml of L-broth, and the suspension was incubated at 25°C for one hour. Aliquots of the cell mixture were plated on L-agar (L-broth with 15 g/l agar) plates containing 50 $\mu$g/ml kanamycin, and the plates were incubated at 25°C. E. coli K12 RV308/pIT337 transformants were verified by selection for kanamycin resistance and by restriction enzyme analysis of the plasmid DNA of the transformants. Plasmid DNA was obtained from the E. coli K12 RV308/pIT337 transformants in substantial accordance with the teaching of Example 2A, but on a smaller scale, and the CsCl-gradient steps were omitted.

B. **Culture of E. coli K12 RV308/pIT337 for Expression of Isopenicillin N Synthetase Activity**

Several isolates of the E. coli K12 RV308/pIT337 transformants prepared in Example 3A were individually inoculated into 5 ml aliquots of L-broth containing 50 $\mu$g/ml kanamycin, and the cultures were incubated in an air-shaker at 25°C until the O.D$_{590}$ was $\sim$0.2 absorbance units. The cultures were then transferred to a 37°C air-shaker and incubated at 37°C for $\sim$6 hours.

After the six-hour, 37°C incubation, one ml of each culture was collected, and the cells were pelleted by centrifugation. The cell pellets were individually washed with 1 ml of 10 mM NaCl and then resuspended in 1.0 ml of IPS extraction buffer (0.05 M Tris-HCl, pH = 8.0; 0.01 M KCl; and 0.01 M $MgSO_4$). The cells were sonicated by six, five-second bursts of sonication delivered by a Sonifier Cell Disruptor, Model W185, Heat Systems-Ultrasonics, Inc., Plainview, Long Island, NY, using the micro tip. The time between bursts of sonication was 60 seconds, and the mixture was kept in an ice-ethanol bath during the procedure. After sonication, the cell mixture was centrifuged to remove debris and then used directly in the assay.

C. Assay for Isopenicillin N Synthetase Activity

The following assay procedure is derived from the procedure of Shen et al., 1984, J. of Antibiotics 37-(9): 1044-1048.

The isopenicillin N synthetase assay reaction was carried out in a total volume of 500 $\mu$l. To start the reaction, 1.0 ml of a solution of 1.4 mM $\delta$-(L-$\alpha$-aminoadipyl)-L-cysteinyl-D-valine and 3.75 mM DTT was allowed to react at room temperature for 30-60 minutes to reduce any dimeric tripeptide to the monomeric form. Fifty $\mu$l of each of the following stock solutions were aliquoted into each assay tube (sterile, glass, disposable 13 x 100 mm tubes): 500 mM Tris-HCl, pH = 7.4; 100 mM KCl; 100 mM $MgSO_4$; 2.0 mM $FeSO_4$; and 6.7 mM ascorbic acid. Next, varying amounts of extract, diluted with water to a volume of 150 $\mu$l, were added. About 100 $\mu$l aliquots of the tripeptide solution were then added to each tube; the addition of the tripeptide starts the reaction. Each tube was vortexed upon addition of the substrate. The reaction mixture vessels were then placed in a gyrotory shaker bath at 250 rpm, with an incubation temperature of 25°C. The reaction time was 45 minutes.

After 45 minutes of reaction, 2 samples of 100 $\mu$l each were withdrawn and dispensed into wells in the bioassay plates, and 100 units of penicillinase A were added to the remainder of the sample. The penicillinase A was obtained from Riker's Laboratories, Inc.; the enzyme is sold in vials of 100,000 units, which were rehydrated to 5.0 mls with $H_2O$. Five $\mu$l (100 units) of the rehydrated pencillinase A were added to each reaction mixture, allowed to react for 5 minutes at room temperature, and then 100 $\mu$l of each

penicillinase A-treated extract were dispensed into the wells of a bioassay plate. This penicillinase A treatment is done to check that the zones on the bioassay plate are due to the presence of a penicillin rather than a cephalosporin or other contaminant.

The penicillin N standard curve was prepared by adding 0.5, 1.0, 2.0, 5.0, 10.0, and 20.0 μg of penicillin N to bioassay wells. The penicillinase A activity was also checked by adding 5 μl of the enzyme preparation to ∿200 μl of 0.2 μg/ml penicillin N.

The bioassay plates were composed of K131 nutrient agar, which is prepared by dissolving 30.5 g BBL Antibiotic Medium #11 (Becton Dickinson & Company, Cockeysville, MD) in 1 liter of deionized water, bringing the solution to a boil, cooling to 70°C, and then autoclaving 35 minutes at 121°C and 15 psi. The plates were seeded with 4 mls of fresh overnight culture of Micrococcus luteus (ATCC 9341) per 700 ml of agar. The M. luteus was grown in K544 nutrient broth, which is composed of: Difco peptone, 5.0 g; Difco yeast extract, 1.5 g; sodium chloride, 3.5 g; dipotassium phosphate (anhydrous), 3.7 g; monopotassium phosphate, 1.3 g; Difco beef extract, 1.5 g, in 1 liter of deionized water -- the solution is brought to a boil, cooled to 25°C, adjusted to a pH = 7.0 with 1 N HCl or 1 N NaOH, and then autoclaved for 20 minutes at 121°C and 15 psi before use. The seeded agar was dispensed into 100 x 15 mm plates, at 15 mls of seeded agar per plate. The wells were prepared by applying suction using a disposable 5 ml pipette; each well was 10 mM in diameter.

After the plates were prepared and the samples were dispensed into the wells, the plates were placed in a 37°C incubator for 18 hours. The assay results are determined by measuring the diameter of the cleared areas around each sample well, which result from the M. luteus being unable to grow when a penicillin is present.

The results of the assay are tabulated below.

TABLE II

| Isopenicillin N Synthetase Activity of Cell Extracts from E. coli K12 RV308/plT337 | |
| --- | --- |
| Sample | Zone Size (mm) |
| 2 μg penicillin N standard | 16 |
| 5 μg penicillin N standard | 18 |
| 10 μg penicillin N standard | 27 |
| 20 μg penicillin N standard | 31 |
| 25 μl E. coli K12 RV308/plT337 cell extract | 10 |
| 50 μl E. coli K12 RV308/plT337 cell extract | 22 |
| 100 μl E. coli K12 RV308/plT337 cell extract | 27 |
| 150 μl E. coli K12 RV308/plT337 cell extract | 29 |
| All penicillinase-treated samples | 0 |
| E. coli K12 RV308/pCZ106 cell extract control | 0 |
| Control reactions without substrate | 0 |

Although the linearity of the assay, as measured by zone size, drops off markedly when zone size increases above 21 mm, the results of the assay clearly indicate that the E. coli K12 RV308/plT337 transformants express isopenicillin N synthetase activity, whereas the E. coli K12 RV308/pCZ106 transformants do not.

The E. coli-produced material is substantially more stable than isopenicillin N synthetase derived from Cephalosporium acremonium. This greater stability was first observed in freeze-thaw experiments. The C. acremonium isopenicillin N synthetase activity is quickly inactivated by refreezing and rethawing, but the E. coli-produced isopenicillin N synthetase activity of the present invention is quite resistant to freezing and thawing.

The greater stability probably results from a difference in processing of the enzyme between C. acremonium and E. coli. For instance, the isopenicillin N synthetase activity isolated from C. acremonium does not appear to have the first two amino-terminal amino acid residues, methionine and glycine, which are encoded in the C. acremonium isopenicillin N synthetase activity-encoding DNA and which are also present in the E. coli produced material of the present invention. As disclosed in Tsunasawa et al., 1985, J. of Biol. Chem. 260(9):538291. E. coli produces a peptidase that cleaves the amino-terminal methionine residue of a protein when the following residue has a relatively small side chain. In the IPS protein, the amino-terminal methionine is followed by a glycine residue, so the amino-terminal methionine is cleaved.

In view of the greater stability and different amino acid residue sequence of the E. coli-produced isopenicillin N synthetase activity, the present invention also comprises a novel protein: E. coli-produced isopenicillin N synthetase.

## Example 4

### Construction of Plasmid pPS20

#### A. Preparation of HindIII-Digested Plasmid pIT335.

Five $\mu$l of the plasmid pIT335 DNA prepared in Example 1B, which correspond to ∿5 $\mu$g of plasmid DNA, were added to and mixed with 5 $\mu$l of 10X HindIII reaction buffer (500 mM NaCl; 500 mM Tris-HCl, pH = 8.0; 100 mM MgCl$_2$; and 1 mg/ml BSA), 5 $\mu$l (∿50 units) of restriction enzyme HindIII, and 35 $\mu$l of H$_2$O. The resulting reaction was incubated at 37°C for four hours. The HindIII-digested plasmid pIT335 DNA was extracted once with phenol and then extracted once with CHCl$_3$. After the extractions, the HindIII-digested plasmid pIT335 DNA was made 0.25 M in NaCl, diluted with two volumes of absolute ethanol, chilled in a dry ice-ethanol bath, and then the precipitated DNA was collected by centrifugation. The ∿5 $\mu$g of HindIII-digested plasmid pIT335 DNA obtained by this procedure were dissolved in 10 $\mu$l of TE buffer and stored at -20°C.

#### B. HindIII Digestion of Plasmid pIT221 and Isolation of the ∿2.7 kb HindIII Restriction Fragment of Plasmid pIT221 that Comprises a Hygromycin Resistance-Conferring Gene

European Patent Application No. 85306765.0 discloses vectors and conditions for transforming Cephalosporium acremonium. Construction flow sheets 1-6 and Examples 1-6 of U.S. Patent Application Serial No. 654,919, incorporated herein by reference, disclose the construction of plasmid pIT221. A restriction site and function map of plasmid pIT221 is provided in Figure 4 of the accompanying drawings.

Plasmid pIT221 was isolated from E. coli K12 JA221/pIT221 in substantial accordance with the procedure of Example 1 of the present application. About 50 $\mu$g of plasmid pIT221 were digested in 100 $\mu$l of 1X HindIII reaction buffer with 100 units of restriction enzyme HindIII in substantial accordance with the procedure of Example 4A. After extracting, precipitating, and redissolving the HindIII-digested plasmid pIT221 DNA in accordance with the procedure of Example 4A, the DNA was loaded onto a 1% agarose gel for electrophoresis. The desired ∿2.7 kb HindIII restriction fragment of plasmid pIT221 that comprises the yeast Saccharomyces cerevisiae phosphoglycerate kinase transcriptional and translational activating sequence and encodes a hygromycin resistance-conferring phosphotransferase enzyme was isolated and purified from the gel and other digestion products in substantial accordance with the procedure of Example 2B.

About 5 $\mu$g of the desired ∿2.7 kb HindIII restriction fragment were isolated by the foregoing method. The purified fragment obtained was dissolved in 10 $\mu$l of TE buffer and stored at -20°C.

#### C. Final Construction of Plasmid pPS20

About 1 $\mu$l of the HindIII-digested plasmid pIT335 DNA prepared in Example 4A and 4 $\mu$l of the ∿2.5 kb HindIII restriction fragment of plasmid pIT221 prepared in Example 4B were ligated in 30 $\mu$l of ligation buffer with 100 units of T4 DNA ligase in substantial accordance with the procedure of Example 2C. The ligated DNA constituted the desired plasmid pPS20. A restriction site and function map of plasmid pPS20 is presented in Figure 5 of the accompanying drawings.

The ∿2.7 kb HindIII restriction fragment could insert into plasmid pIT335 in either of two orientations, so the ligated DNA also constituted another plasmid, designated plasmid pPS20.1. Plasmid pPS20.1 is functionally equivalent to plasmid pPS20 and differs from plasmid pPS20 only with respect to the orientation of the ∿2.7 kb HindIII restriction fragment.

#### D. Construction of E. coli K12 JA221/pPS20 and Isolation of Plasmid pPS20 DNA

A 50 ml culture of E. coli K12 JA221 (NRRL B-15211) in L-broth was grown to an O.D.$_{590}$ of ∿0.2. The culture was chilled on ice for ten minutes, and the cells were collected by centrifugation. The cell pellet was resuspended in 25 ml of cold 100 mM CaCl$_2$ and incubated on ice for 25 minutes. The cells were once again pelleted by centrifugation, and the pellet was resuspended in 2.5 ml of cold 100 mM CaCl$_2$ and

incubated on ice overnight.

Two hundred $\mu$l of this cell suspension were mixed with the ligated DNA prepared in Example 4C and incubated on ice for 20 minutes. The mixture was then incubated at 40°C for 2 minutes, followed by a 10 minute incubation at room temperature. Three ml of L-broth were added to the cell mixture, and then the cells were incubated in an air-shaker at 37°C for two hours.

Aliquots of the cell mixture were plated on L-agar (L-broth with 15 g/l agar) plates containing 100 $\mu$g/ml ampicillin, and the plates were then incubated at 37°C. E. coli K12 JA221/pPS20 transformants were verified by restriction enzyme analysis of the plasmid DNA of the ampicillin-resistant transformants. Plasmid DNA was obtained from the E. coli K12 JA221/pPS20 and E. coli K12 JA221/pPS20.1 transformants in substantial accordance with the procedure of Example 1, but on a smaller scale, and the CsCl gradient steps were omitted.

## Example 5

### Construction of Plasmid pPS21

#### A. NcoI Digestion and Klenow Treatment of Plasmid pIT335 DNA and Isolation of the Resulting ~0.85 kb Fragment that Encodes a Cephalosporium acremonium Transcriptional and Translational Activating Sequence

Approximately 50 $\mu$l, corresponding to 50 $\mu$g, of the plasmid pIT335 DNA prepared in Example 1 were added to and mixed with 10 $\mu$l 10X BamHI buffer, 5 $\mu$l (~50 units) restriction enzyme NcoI, and 35 $\mu$l of $H_2O$. The resulting reaction was incubated at 37°C for four hours. The reaction mixture was then made 0.25 M in NaCl, diluted with two volumes of absolute ethanol, chilled for 10 minutes in a dry ice-ethanol bath, and centrifuged to pellet the precipitated DNA.

The NcoI-digested plasmid pIT335 DNA pellet was dissolved in 50 $\mu$l of 1X Klenow buffer (40 mM $KPO_4$, pH = 7.5; 6.6 mM $MgCl_2$; 1.0 mM 2-mercaptoethanol; 33 $\mu$M dATP; 33 $\mu$M dCTP; 33 $\mu$M dGTP; and 33 $\mu$M TTP). Two $\mu$l (~10 units, New England Biolabs) of the large fragment of E. coli DNA polymerase I, known as Klenow, were added to and mixed with the DNA, and the resulting reaction was incubated at 16°C for one hour. The reaction was terminated by a buffered phenol extraction.

The NcoI-digested, Klenow-treated plasmid pIT335 DNA was then loaded onto a 1% agarose gel for electrophoresis. The ~0.85 kb restriction fragment that comprises the Cephalosporium acremonium transcriptional and translational activating sequence of the IPS gene was isolated from the gel and purified in substantial accordance with Example 2B. About 4 $\mu$g of the desired fragment were obtained and suspended in 10 $\mu$l of TE buffer.

#### B. Construction of Intermediate Plasmid pPS19

One $\mu$g of plasmid pIT221 DNA was dissolved in five $\mu$l of 10X XmaI buffer (250 mM NaCl; 60 mM Tris-HCl, pH = 7.5; 60 mM $MgCl_2$; 60 mM 2-mercaptoethanol; and 1 mg/ml BSA), 43 $\mu$l of $H_2O$ and 2 $\mu$l (~10 units) of restriction enzyme XmaI. The resulting reaction was incubated at 37°C for four hours. The reaction was terminated by a phenol extraction. After further extracting the XmaI reaction mixture with $CHCl_3$, the reaction mixture was made 0.25 M in NaCl, diluted with 2 volumes of absolute ethanol, chilled for 10 minutes in a dry ice-ethanol bath, and the precipitated, XmaI-digested plasmid pIT221 DNA was pelleted by centrifugation.

The XmaI-digested plasmid pIT221 DNA was redissolved in 100 $\mu$l of 1X ligation buffer containing 500 units of T4 DNA ligase. The ligation reaction was incubated at 12°C for ~16 hours and then used to transform E. coli K12 JA221 in substantial accordance with the procedure of Example 4D. The ampicillin-resistant, plasmid pPS19 transformants were identified by restriction enzyme analysis of the plasmid DNA of the transformants. Plasmid pPS19 DNA was prepared in substantial accordance with the procedure of Example 1. A restriction site and function map of plasmid pPS19 is presented in Figure 6 of the accompanying drawing.

#### C. BamHI Digestion and Klenow Treatment of Plasmid pPS19 DNA and Isolation of the ~7.7 kb Fragment

Fifty $\mu$g of plasmid pPS19 DNA were digested with restriction enzyme BamHI and treated with Klenow in substantial accordance with the procedure of Example 4A, except that BamHI restriction enzyme, rather

## EP 0 200 425 B1

than NcoI restriction enzyme, was used to digest the plasmid pPS19 DNA. The BamHI-digested, Klenow-treated plasmid pPS19 DNA was loaded onto a 1% agarose gel, and the ∿7.7 kb fragment was isolated and purified in substantial accordance with the procedure of Example 2B. About 5 μg of the desired fragment were obtained, dissolved in 10 μl of TE buffer, and stored at -20°C.

### D. Final Construction of Plasmid pPS21

Two μl of the ∿0.85 kb fragment prepared in Example 5A were ligated to two μl of the ∿7.7 kb fragment prepared in Example 5C in 30 μl of 1X ligation buffer containing 500 units of T4 DNA ligase. The ligation reaction was incubated at 12°C for 16 hours, and the ligated DNA constituted the desired plasmid pPS21 DNA.

### E. Construction of E. coli JA221/pPS21

The ligated DNA prepared in Example 5D was used to transform E. coli K12 JA221 in substantial accordance with the procedure of Example 4D. The ampicillin-resistant transformants were screened for the presence of plasmid pPS21 by restriction enzyme analysis of the plasmid DNA of the transformants. Because the ∿0.8 kb fragment could insert into the ∿7.7 kb fragment of plasmid pPS19 in either one of two orientations, and because only one orientation correctly positions the Cephalosporium acremonium transcriptional and translational activating sequence for expression of the hygromycin resistance-conferring gene, only about half of the transformants were the desired E. coli K12 JA221/pPS21. One such E. coli K12 JA221/pPS21 transformant was used to prepare plasmid pPS21 DNA in substantial accordance with the procedure of Example 1.

### Example 6

### Genetic Transformation of Cephalosporium acremonium with Plasmids pPS20 and pPS21

European Patent Publication No. 0177243 discloses and claims the following transformation procedure.

### A. Cephalosporium acremonium Strains

The preferred Cephalosporium strain for transformation is obtained from the American Type Culture Collection, Rockville, Maryland, under the accession number ATCC 11550. Other Cephalosporium strains or any commercial strains derived from ATCC 11550 by mutation, selection, or genetic breeding for the purpose of improved production of cephalosporin C are also suitable for use in preparing transformants with the vectors and plasmids of the present invention.

### B. Preparation of Inoculum for Cell Culture

To genetically transform Cephalosporium acremonium cells efficiently, it is necessary to remove the cell walls to form stable protoplasts. In the preparation of such protoplasts, it is highly advantageous to begin with a uniform inoculum. Otherwise, preparation of cells in culture is not reproducible and time is lost by attempts to prepare C. acremonium protoplasts from unsuitable or inadequate amounts of cells.

### C. Preparation of Uniform Inoculum for Cell Culture

An ampoule of spores (approximately $10^9$ conidia in 1.5 ml of preservation menstrum: 5% lactose, 10% glycerol, and 0.1% Tween 80), either lyophilized or taken from liquid nitrogen storage and thawed at room temperature, are diluted in 5 ml of sterile saline. About 0.1 ml of this suspension is used to inoculate each of approximately 50 slants containing 20 ml of Trypticase®-Soy Agar (BBL™, Division of Becton, Dickinson & Company, Cockeysville, Maryland 21030) medium. Before inoculation, the medium is allowed to dry until surface moisture is no longer visible. Inoculated slants are incubated for about four days at 25°C. About 10 ml of preservation menstrum are added to the mycelial growth which covers the surface of the medium in each slant. The slants are vortexed to suspend the conidia, and the conidial suspension from each slant is pooled and 10 ml aliquots frozen at -80°C. The frozen conidial suspension slowly loses viability and should not be used after about three months of storage at -80°C.

24

D. Growth of Cells for Preparation of Protoplasts

Approximately 106 ml of aqueous medium in a 500 ml shake flask are inoculated with cells from the 10 ml of frozen conidial suspension prepared in Example 6C. Cells are obtained by centrifugation (10 min X 2600 rpm), and then directly suspended in the aqueous culture medium * . Decantation of the supernatant is necessary prior to suspension, because the lactose and glycerol adversely affect the growth of cells. The flask containing the suspended cells is placed on a gyrotory water bath shaker and incubated at 29-30°C for 24 hours at 285 rpm with a 1 inch throw. The recommended temperature of 29-30°C in the culturing step is especially preferred for preparing transformable protoplasts, but lower temperatures of about 25°C are also suitable. Those familiar with the art will recognize that the 29-30°C is different from the temperature (25°C) preferred for culturing Cephalosporium acremonium for purposes of antibiotic production.

E. Preparation of Cephalosporium Protoplasts

Cells from a 24 hour culture are harvested by suction filtration (Whatman #1 paper in a Buchner funnel) and suspended in McIlvaine's Buffer, pH = 7.1, (0.1 M citric acid and 0.2 M dibasic sodium phosphate) to which dithiothreitol has been added to a concentration of 0.01 M. Sufficient buffer is added to obtain a final cell concentration of 1 g (weighed after suction filtration) of cell mass per 20 ml of buffer. The cell suspension is placed on a gyrotory water bath shaker in a 50 ml shake flask and incubated at 29-30°C for 90 minutes at 140 rpm with 1 inch throw. Dithiothreitol-treated cells are washed with water and then resuspended in enzyme solution (25 mg/ml of beta-glucuronidase from Sigma Chemical Company, in McIlvaine's buffer, pH = 6.35, and supplemented with 0.8 M NaCl and 0.02 M $MgSO_4$). The final cell concentration is 1 g of treated cell mass per 10 ml of enzyme solution. The cell suspension is then placed on a gyrotory water bath shaker at 29-30°C for 3 hours at 120 rpm with a 1 inch throw. The suspension of protoplasts is diluted with 4 volumes of washing solution (0.8 M NaCl and 0.02 M $MgSO_4$) and then gravity filtered through two layers of paper towels. The filtrate containing the protoplasts is centrifuged at room temperature for 5 minutes at 2600 rpm. The supernatant is decanted, and the pellet of protoplasts is suspended in 10 ml of washing solution. After repeating the washing procedure twice, the protoplasts are resuspended in sufficient 0.8 M NaCl to achieve a concentration of 2 to 3 x $10^8$ protoplasts per ml, by hemacytometer count.

F. Transformation Procedure

For each plasmid to be transformed, a 1 ml suspension of Cephalosporium protoplasts (2 to 3 x $10^8$ per ml) in 0.8 M NaCl is added to 0.005 ml of freshly distilled DMSO and then made 80 mM in $CaCl_2$. About 20 μg of transforming plasmid, either pPS20 or pPS21, depending on the transformation, and polyethylene glycol 4000 (Baker, >20% w/v in water) are added to the suspension of protoplasts to achieve a mixture with a volume of 10 ml. The mixture is incubated for 10 minutes at room temperature and then centrifuged at 700 rpm for 5 minutes, which is followed by a 2500 rpm centrifugation for 10 minutes. The pellet of protoplasts is suspended in 1 ml of 0.8 M NaCl. Aliquots (0.1 ml) are delivered to the surface of Trypticase-Soy Agar medium (BBL) that has been enriched with 10.8% sucrose to osmotically stabilize the protoplasts. After the petri plates are incubated at 15°C for 24 hours, 4 ml of liquified agar (0.41% w/v, at 42°C) containing 0.8 M sodium chloride and sufficient hygromycin to achieve a final concentration of 100 μg/ml are added to each petri dish. After the overlay has solidified, the petri plates are then incubated at 25°C in a humidified chamber. Although transformant colonies of sufficient size to subculture are present 12 days

*Aqueous culture medium was prepared as follows: one hundred ml of solution A are dispensed into a 500 ml shake flask; the flask is covered with a commercial closure and is autoclaved at 121°C for 20 minutes. Two ml of solution B and 4 ml of solution C are then added to solution A to prepare the aqueous culture medium.
Solution A: Sucrose, 36 g/L; L-asparagine, 7.5 g/L; $KH_2PO_4$, 15 g/L; $K_2HPO_4$, 21 g/L; $Na_2SO_4$, .75 g/L, $MgSO_4$ $7H_2O$; .18 g/L; $CaCl_2$, .06 g/L; salts solution, 1 ml/L; natural pH.
Salts solution:
$Fe(NH_4)(SO_4)_2.6H_2O$, 15 g/L; $MnSO_4.4H_2O$, 3 g/L: $ZnSO_4.7_2O$, 3 g/L; $CuSO_4.5H_2O$, 0.8 g/L).
Solution B: Glucose, 108 g/L (autoclaved at 121°C, 30 minutes)
Solution C: Sucrose, 25 g/L; corn steep liquor (4% w/v nitrogen), 12.5 ml; ammonium acetate, 5.5 g/L; $CaCO_3$, 5 g/L; pH adjusted to 6.5 with KOH; and autoclaved at 121°C for 20 minutes.

25

after transformation, slower growing transformants may take as long as 60 days to achieve a suitable size for subculture. Abortive transformants are easily distinguished from stable transformants, because abortive transformants fail to grow upon subculture to fresh medium containing 100 µg/ml of hygromycin.

## G. Analysis of Cephalosporium acremonium/pPS20 and C. acremonium/pPS21 Transformants

Cephalosporium acremonium/pPS20 transformants express significantly higher levels of isopenicillin N synthetase activity than do C. acremonium transformants of control plasmids, such as plasmid pIT221. This higher level of activity results in an increased ability of the transformants to make isopenicillin N, whether in fermentation or in cell extracts of the C. acremonium/pPS20 transformants.

Cephalosporium acremonium/pPS21 transformants are hygromycin-resistant, which indicates the functionality of the C. acremonium transcriptional and translational activating sequence of the present invention.

Example 7

Construction of Plasmids pPS21A, pPS22, pPS23, pPS23.1, pPS24, pPS25, and pPS25.1

A. Construction of Intermediate Plasmids pPS23 and pPS23.1

(i) Preparation of BamHI-digested plasmid pUC8.

About 5 µg of plasmid pUC8 (obtained from Pharmacia P-L Biochemicals) were dissolved in 5 µl of 10X BamHI reaction buffer and 40 µl of $H_2O$. About 5 µl (50 units) of restriction enzyme BamHI were added to the solution of DNA, and the resulting reaction was incubated at 37°C for two hours. The reaction was terminated by extraction with buffered phenol, followed by extraction with chloroform. The BamHI-digested plasmid pUC8 DNA was precipitated by adjusting the NaCl concentration to 0.25 M, adding 2 volumes of ethanol, and chilling at -70°C for 10 minutes. The BamHI-digested plasmid pUC8 DNA was collected by centrifugation and resuspended in 5 µl of $H_2O$.

(ii) Isolation of the ∿0.85 kb NcoI restriction fragment of plasmid pIT335.

About 10 µg of plasmid pIT335 were dissolved in 5 µl of 10X BamHI buffer and 40 µl of $H_2O$. About 5 µl (50 units) of restriction enzyme NcoI were added to the solution of DNA, and the resulting reaction was incubated at 37°C for two hours. The reaction mixture was then loaded onto a 1% agarose gel, and the desired ∿0.85 kb NcoI restriction fragment that comprises the transcription and translation activating sequence of the IPS gene was isolated in substantial accordance with the procedure of Example 2B. About 1 µg of the desired fragment was obtained and suspended in 5 µl of $H_2O$.

(iii) Preparation of the linker used in the construction of plasmid pPS23.

The single-strands of the following linker were synthesized using an automated DNA synthesizer:

$$5'-CATGAAGAAG-3'$$
$$| \; | \; | \; | \; | \; |$$
$$3'-TTCTTCCTAG-5'$$

About 75 picomoles of each single strand of the linker were individually dissolved in 22.5 µl of $H_2O$ and 2.5 µl of ligase buffer. About 1 µl (10 units) of T4 DNA kinase (Bethesda Research Laboratories) was added to each solution of single-stranded DNA, and the reactions were incubated at 37°C for 10 minutes. Following the kinase reaction, the reaction mixtures were incubated at 70°C for 15 minutes. Then, to anneal the single-stranded DNA to form the linker, the two reaction mixtures were pooled, incubated at 65°C for 10 minutes, incubated at room temperature for 2 hours, and then incubated at 4°C overnight.

(iv) Final Construction of plasmids pPS23 and pPS23.1.

One µl of the BamHI-digested plasmid pUC8 DNA was added to a mixture of 4 µl of the ∿0.85 kb NcoI restriction fragment of plasmid pIT335 and 10 µl of the annealed linker. About 4 µl of 10X ligase buffer, 2 µl (500 units) T4 DNA ligase, and 29 µl of $H_2O$ were added to the mixture of DNA, and the resulting reaction was incubated at 4°C overnight. The ligated DNA constituted the desired plasmids pPS23 and pPS23.1.

A 50 ml culture of E. coli K12 JM109, available from Pharmacia P-L Biochemicals, in L-broth was grown to an $O.D._{590}$ of approximately 0.5 absorbance units. The culture was chilled on ice for ten minutes, and the cells were collected by centrifugation. The cell pellet was resuspended in 25 ml of cold 100 mM $CaCl_2$ and

incubated on ice for 25 minutes. The cells were once again pelleted by centrifugation, and the pellet was resuspended in 2.5 ml of cold 100 mM $CaCl_2$ and incubated on ice overnight.

Two hundred $\mu l$ of this cell suspension were mixed with the ligated DNA prepared above and incubated on ice for 20 minutes. At the end of this period, the cells were placed in a water bath at $42^\circ$ C for 2 minutes and then returned to the ice for an additional 10 minutes. The cells were collected by centrifugation and resuspended in one ml of L-broth and incubated at $37^\circ$ C for 2 hours.

Aliquots of the cell mixture were plated on L-agar (L-broth with 15 grams per liter agar) plates containing 100 $\mu g$ ampicillin/ml, 40 $\mu g$ X-gal/ml, and 40 $\mu g$ IPTG/ml. The plates were incubated at $37^\circ$ C overnight. Colonies that contain a plasmid without an insert, such as E. coli K12 JM109/pUC8, appear blue on these plates. Colonies that contain a plasmid with an insert, such as E. coli K12 JM109/pPS23, are white. Several white colonies were selected and screened by restriction analysis of their plasmid DNA for the presence of the ∿0.85 kb BamHI restriction fragment containing the IPS activating sequence. Plasmid DNA was obtained from the E. coli K12 JM109/pPS23 and E. coli K12 JM109/pPS23.1 cells in substantial accordance with the teaching of Example 2A.

## B. Isolation of the ∿0.85 kb BamHI Restriction Fragment of Plasmid pPS23

About 50 $\mu g$ of plasmid pPS23 DNA were dissolved in 15 $\mu l$ of 10X BamHI reaction buffer and 125 $\mu l$ of $H_2O$. About 10 $\mu l$ (100 units) of restriction enzyme BamHI were added to the solution of DNA, and the resulting reaction was incubated at $37^\circ$ C for two hours. The BamHI-digested plasmid pPS23 DNA was loaded onto a 1% agarose gel, and the ∿0.85 kb BamHI restriction fragment that comprises the activating sequence of the IPS gene was isolated in substantial accordance with the procedure of Example 2B. About 5 $\mu g$ of the desired fragment were obtained and suspended in 10 $\mu l$ of $H_2O$.

## C. Preparation of BamHI-Digested Plasmid pPS19 DNA

About 5 $\mu g$ of plasmid pPS19 DNA were dissolved in 10 $\mu l$ 10X BamHI reaction buffer and 35 $\mu l$ of $H_2O$. About 5 $\mu l$ (50 units) of restriction enzyme BamHI were added to the solution of plasmid pPS19 DNA, and the resulting reaction was incubated at $37^\circ$ C for two hours. The reaction mixture of BamHI-digested plasmid pPS19 DNA was extracted once with buffered phenol and then extracted twice with phenol. The DNA was then precipitated, collected by centrifugation and resuspended in 10 $\mu l$ of $H_2O$.

## D. Final Construction of Plasmids pPS21A, pPS22, pPS24, pPS25, and pPS25.1

About 1 $\mu l$ of the ∿0.86 kb BamHI restriction fragment was added to 1 $\mu l$ of the BamHI-digested plasmid pPS19 DNA, 3 $\mu l$ 10X ligase buffer, 2 $\mu l$ T4 DNA ligase, and 23 $\mu 1$ of $H_2 0$. The resulting ligation reaction was incubated at $15^\circ$ C overnight. The ligated DNA constituted the desired plasmids pPS21A, pPS22, pPS24, pPS25 and pPS25.1.

The ligated DNA was used to transform E. coli K12 C600, a strain available from the American Type Culture Collection, Rockville, MD 20852, under the accession number ATCC 33525, in substantial accordance with the procedure of Example 7A(iv). The transformed cells were plated on L-agar plates containing 100 $\mu g$/ml ampicillin, and the plates were incubated at $37^\circ$ C overnight.

Individual colonies were picked from the transformation plates, cultured, and used to prepare plasmid DNA. The plasmid DNA was analyzed by restriction enzyme analysis. The following chart demonstrates the appropriate restriction enzyme digests that can be used to distinguish the plasmids.

| Plasmid | Enzyme | Size of Fragments (in kb) |
|---------|--------|---------------------------|
| pPS19 | BamHI | 7.62 and 0.23 |
| | PstI | 5.15, 1.85, and 0.85 |
| pPS21A | BamHI | 7.62 and 0.85 |
| | PstI | 5.15, 1.85, 0.99, and 0.49 |
| pPS22 | BamHI | 7.62 and 0.85 |
| | PstI | 5.15, 1.85, 0.94, and 0.54 |
| pPS24 | BamHI | 7.62 |
| | PstI | 7.62 |
| pPS25 and pPS25.1 | BamHI | 5.15, 1.85, 0.99, and 0.72 |
| | PstI | 7.62, 0.85, and 0.23 |

Restriction site and function maps of plasmids pPS21A and pPS25 are respectively presented in Figures 8 and 9 of the accompanying drawings.

Plasmids pPS21A, pPS25.1, and pPS25 were used to transform Cephalosporium acremonium in substantial accordance with the procedure of Example 6. The C. acremonium/pPS21A, C. acremonium/pPS25.1, and C. acremonium/pPS25 transformants were hygromycin-resistant. Plasmids pPS22 and pPS24 were also used to transform C. acremonium, but these plasmids transformed C. acremonium to hygromycin resistance at a much lower frequency than did plasmids pPS21A, pPS25.1, and pPS25, presumably because plasmids pPS22 and pPS24 must integrate into the C. acremonium genome in the proper position for a genomic C. acremonium activating sequence to drive expression of the hygromycin resistance-conferring gene.

Example 8

Construction of Plasmids pPS28 and pPS29

About 20 µl of plasmid pPS21A DNA were dissolved in 10 µl 10X PstI reaction buffer (1.0M NaCl; 100 mM Tris-HCl, pH = 7.5; 100 mM MgCl₂; and 1 mg/ml BSA) and 88 µl of H₂O. About 2 µl (150 units) of restriction enzyme PstI were added to the solution of DNA, and the reaction was incubated at 37°C for 4 minutes, and then, the reaction was terminated by incubation at 70°C for 10 minutes. The partially PstI-digested plasmid pPS21A DNA was loaded onto an agarose gel, and after electrophoresis and staining of the gel, the following fragments were observed: 8.5 kb (linearized plasmid); 8.0 kb; 7.5 kb; 7.0 kb; 6.6 kb; 6.1 kb; 5.2 kb; 3.3 kb; 2.3 kb; 1.9 kb; 1.5 kb; 1.0 kb; and 0.5 kb. The ∿6.6 kb and ∿6.1 kb PstI restriction fragments were individually isolated in substantial accordance with the procedure of Example 2B; about 0.5 µg of each fragment were recovered.

The ∿6.6 kb PstI restriction fragment was dissolved in 3 µl 10X ligase buffer and 25 µl of H₂O. About 2 µl of T4 DNA ligase were added to the solution of DNA, and the resulting reaction was incubated at 15°C overnight. The ligated DNA constituted the desired plasmid pPS28 DNA, which was used to transform E. coli K12 C600 in substantial accordance with the procedure of Example 7. In a similar fashion, the ∿6.1 kb PstI restriction fragment was circularized by ligation to yield plasmid pPS29, which was also transformed into E. coli K12 C600. Restriction site and function maps of plasmids pPS28 and pPS29 are respectively presented in Figures 10 and 11 of the accompanying drawings.

Plasmids pPS28 and pPS29 were used to transform Cephalosporium acremonium in substantial accordance with the procedure of Example 6. The C. acremonium/pPS28 and C. acremonium/pPS29 transformants exhibited the hygromycin-resistant phenotype, and the plasmid pPS28 and plasmid pPS29 DNA transformed the C. acremonium to hygromycin resistance at high frequency.

Example 9

Construction of Plasmids pPS26 and pPS26.1

About 20 µg of plasmid pPS21A were dissolved in 10 µl 10X HindIII reaction buffer and 85 µl of H₂O. About 5 µl (50 units) of restriction enzyme HindIII were added to the solution of DNA, and the resulting reaction was incubated at 37° C for two hours. The HindIII-digested plasmid pPS21A DNA was loaded onto a 1% agarose gel and electrophoresed until the ∿3.45 kb, ∿3.16 kb, ∿1.2 kb, and ∿0.69 kb HindIII restriction fragments were clearly separated on the gel. The ∿3.45 kb HindIII restriction fragment was isolated in substantial accordance with the procedure of Example 2B. About 5 μg of the desired ∿3.45 kb HindIII restriction fragment were obtained and suspended in 10 μl of H₂O.

About 2 μl of the ∿3.45 kb HindIII restriction fragment of plasmid pPS21A were added to 1 μl of the HindIII-digested plasmid pIT335 prepared in Example 4A, 3 μl 10X ligase buffer, 22 μl of H₂O, and 2 μl of T4 DNA ligase. The resulting ligation reaction was incubated at 15° C overnight. The ligated DNA constituted the desired plasmids pPS26 and pPS26.1. The ligated DNA was used to transform E. coli K12 C600 in substantial accordance with the procedure of Example 7. The E. coli K12 C600/pPS26 and E. coli K12 C600/pPS26.1 transformants were identified by their ampicillin-resistant phenotype and by restriction enzyme analysis of their plasmid DNA. A restriction site and function map of plasmid pPS26 is presented in Figure 12 of the accompanying drawings.

Plasmids pPS26 and pPS26.1 were used to transform Cephalosporium acremonium in substantial accordance with the procedure of Example 6. Plasmids pPS26 and pPS26.1 transformed C. acremonium to hygromycin resistance at high frequency, and the C. acremonium/pPS26 and C. acremonium/pPS26.1 transformants produced significantly more isopenicillin N, as measured by zones of inhibition of growth of Micrococcus luteus, than their untransformed counterparts.

## Example 10

### Construction of Plasmids pPS30, pPS30.1, pPS31, and pPS31.1

### A. Isolation of the ∿3.7 XmaI Restriction Fragment of Plasmid pPS6

Plasmid pPS6 is disclosed in Example 13, pages 72-75, of European Patent Publication No.0177243. About 10 μg of plasmid pPS6 were dissolved in 20 μl 10X XmaI reaction buffer (250 mM NaCl; 100 mM Tris-HCl, pH = 7.5; 100 mM MgCl₂; 100 mM 2-mercaptoethanol; and 1 mg/ml BSA) and 165 μl of H₂0. About 15 μl (30 units) of restriction enzyme XmaI were added to the solution of plasmid pPS6 DNA, and the resulting reaction was incubated at 37° C for four hours.

The XmaI-digested plasmid pPS6 DNA was loaded onto a 1% agarose gel and electrophoresed until the ∿3.7 kb XmaI restriction fragment was clearly separated from the other digestion product. The ∿3.7 kb XmaI restriction fragment was then isolated in substantial accordance with the procedure of Example 2B. About 5 μg of the desired ∿3.7 kb XmaI restriction fragment were obtained and suspended in 10 μl of H₂O.

### B. Final Construction of Plasmids pPS30 and pPS30.1

About 1 μg of plasmid pPS21A DNA was dissolved in 2 μl 10X XmaI reaction buffer and 6 μl of H₂O. About 2 μl (6 units) of restriction enzyme XmaI were added to the solution of plasmid pPS21A DNA, and the resulting reaction was incubated at 37° C for four hours. The reaction was terminated by extraction with buffered phenol, followed by two extractions with chloroform. The reaction mixture was then precipitated, collected by centrifugation, and resuspended in 23 μl of H₂O.

About 2 μl of the ∿3.7 kb XmaI restriction fragment of plasmid pPS6, 3 μl of 10X ligase buffer, and 2 μl of T4 DNA ligase were added to the solution of XmaI-digested plasmid pPS21A DNA, and the resulting ligation reaction was incubated at 15° C overnight. The ligated DNA constituted the desired plasmids pPS30 and pPS30.1, which differ only with respect to the orientation of the ∿3.7 kb XmaI restriction fragment.

The ligated DNA was used to transform E. coli K12 C600 in substantial accordance with the procedure of Example 7. The E. coli K12 C600/pPS30 and E. coli K12 C600/pPS30.1 transformants were identified by their ampicillin-resistant phenotype and by restriction enzyme analysis of their plasmid DNA.

Plasmids pPS30 and pPS30.1 were also used to transform Cephalosporium acremonium. The C. acremonium/pPS30 and C. acremonium/pPS30.1 transformants were resistant to hygromycin.

### C. Final Construction of Plasmids pPS31 and pPS31.1

Plasmids pPS31 and pPS31.1 were constructed and then transformed into E. coli K12 C600 and Cephalosporium acremonium in substantial accordance with the procedure of Example 10B, with the exception that plasmid pPS29, rather than plasmid pPS21A, was used as starting material in the construction.

Example 11

Construction of Plasmid pPS27

A. Construction of Plasmid pIT336

About 1 μg of plasmid pUC8 was dissolved in 2 μl 10X BamHI reaction buffer and 16 μl of H₂O. About 2 μl (20 units) of restriction enzyme BamHI were added to the solution of plasmid pUC8 DNA, and the resulting reaction was incubated at 37°C for two hours. The BamHI-digested plasmid pUC8 DNA was precipitated, collected by centrifugation, and resuspended in 2 μl 10X SalI reaction buffer (1.5 M NaCl; 60 mM Tris-HCl, pH=7.9; 60 mM MgCl₂; 60 mM 2-mercaptoethanol; and 1 mg/ml BSA) and 16 μl of H₂O. About 2 μl (20 units) of restriction enzyme SalI were added to the solution of BamHI-digested plasmid pUC8 DNA, and the resulting reaction was incubated at 37°C for two hours. The reaction was terminated by extraction with phenol, followed by two extractions with chloroform. The SalI-BamHI-digested plasmid pUC8 DNA was precipitated, collected by centrifugation, and resuspended in 5 μl of H₂O.

About 10 μg of plasmid pIT335 were dissolved in 10 μl 10X BamHI reaction buffer and 80 μl of H₂O. About 5 μl (50 units) each of restriction enzymes XhoI and BamHI were added to the solution of plasmid pIT335 DNA, and the resulting reaction was incubated at 37°C for two hours. The reaction mixture was then loaded onto a 1% agarose gel and electrophoresed until the ~1.4 kb BamHI-XhoI restriction fragment was separated from the other reaction products, which were 5.6 kb, 1.3 kb, and 0.01 kb fragments. The ~1.4 kb BamHI-XhoI restriction fragment, which comprises the transcription termination and mRNA polyadenylation and processing signals of the IPS gene, was isolated in substantial accordance with the procedure of Example 2B. About 2 μg of the desired fragment were obtained and suspended in 5 μl of H₂O.

The 5 μl of SalI-BamHI digested plasmid pUC8 were added to 2 μl of the ~1.4 kb BamHI-XhoI restriction fragment of plasmid pIT335, 3 μl 10X ligase buffer, 18 μl of H₂O, and 2 μl of T4 DNA ligase. The resulting reaction was incubated at 15°C overnight. SalI and XhoI overlaps are compatible for ligation, but once ligated, neither SalI nor XhoI will cleave the DNA at the junction. The ligated DNA constituted the desired plasmid pIT336 and was used to transform E. coli K12 RR1ΔM15, available from the NRRL under the accession number NRRL B-15440, in substantial accordance with the procedure of Example 7A(iv). The transformed cells were plated on L-agar plates containing 100 μg/ml ampicillin, 40 μg/ml X-gal, and 40 μg/ml IPTG. Colonies that failed to indicate the blue color on the transformation plates were cultured, used to prepare plasmid DNA, and the plasmid DNA was analyzed by restriction enzyme analysis to identify the E. coli K12 RR1ΔM15/pIT336 transformants. A restriction site and function map of plasmid pIT336 is presented in Figure 14 of the accompanying drawings.

B. Construction of Plasmid pPS35

About 2 μg of plasmid pIT336 were dissolved in 2 μl 10X PstI reaction buffer and 17 μl of H₂O. About 1 μl (10 units) of restriction enzyme PstI was added to the solution of pIT336 DNA, and the resulting reaction was incubated at 37°C for 2 hours. The reaction was terminated by extraction with phenol, followed by two extractions with chloroform. The PstI-digested plasmid pIT336 DNA was then precipitated, collected by centrifugation, and resuspended in 86 μl of H₂O.

About 10 μl of 10X ligase buffer and 4 μl of T4 DNA ligase were added to the solution of PstI-digested plasmid pIT336 DNA, and the resulting reaction was incubated at 15°C overnight. The ligated DNA constituted the desired plasmid pPS35 and was used to transform E. coli K12 JA221, available from the NRRL under the accession number NRRL B-15211, in substantial accordance with the procedure of Example 7A(iv). The transformed cells were plated on L-agar plates containing 100 μg/ml ampicillin. Several ampicillin resistant colonies were isolated and used to prepare plasmid DNA. The desired E. coli K12 JA221/pPS35 transformants were identified by restriction enzyme analysis of their plasmid DNA. A restriction site and function map of plasmid pPS35 is presented in Figure 15 of the accompanying drawings.

C. Final Construction of Plasmid pPS27

About 2 μg of plasmid pPS35 were dissolved in 2 μl 10X HindIII reaction buffer and 17 μl of H₂O. About 1 μl (10 units) of restriction enzyme HindIII was added to the solution of plasmid pPS35 DNA, and the resulting reaction was incubated at 37°C for two hours. The reaction was terminated by extraction with phenol, followed by two extractions with chloroform. The HindIII-digested plasmid pPS35 DNA was then precipitated, collected by centrifugation, and resuspended in 3 μl 10X ligase buffer and 23 μl of H₂O.

About 2 $\mu$l of the ∿2.3 kb HindIII restriction fragment of plasmid pPS29, which was prepared and isolated in substantial accordance with the procedure of Example 9 using plasmid pPS29 as starting material instead of plasmid pPS21A, and which comprises the hygromycin resistance-conferring gene driven by the activating sequence of the IPS gene, and 2 $\mu$l of T4 DNA ligase were added to the solution of HindIII-digested plasmid pPS35 DNA. The resulting ligation reaction was incubated at 15°C overnight. The ligated DNA constituted the desired plasmid pPS27 and was used to transform E. coli K12 JA221 in substantial accordance with the procedure of Example 11B.

The ampicillin-resistant transformants were cultured and used to prepare plasmid DNA, which was analyzed by restriction enzyme analysis to identify the desired E. coli K12 JA221/pPS27 transformants. Only one orientation of the inserted ∿3.45 kb HindIII restriction fragment produces the desired plasmid pPS27. A restriction site and function map of plasmid pPS27 is presented in Figure 12 of the accompanying drawings.

Plasmid pPS27 transforms Cephalosporium acremonium to a hygromycin-resistant phenotype at high frequency. C. acremonium/pPS27 transformants are prepared in substantial accordance with the procedure of Example 6.

## Claims

1. A DNA compound selected from
   (a) the ∿1.5 kb NcoI-BamHI restriction fragment of plasmid pIT335 obtainable from E. coli K12 JA221/pIT335 (NRRL B-15960), and
   (b) DNA sequences that hybridise to the foregoing DNA compound under stringent conditions and which code for an isopenicillin N synthetase.

2. The DNA compound of Claim 1 which is the ∿1.5 kb NcoI-BamHI restriction fragment of plasmid pIT335.

3. The DNA compound of Claim 1 wherein the sequence of the coding strand is

```
5'-ATG GGT TCC GTT CCA GTT CCA GTG GCC AAC GTC CCC CGA
ATC GAT GTC TCG CCC CTA TTC GGC GAT GAC AAG GAG AAG AAG
CTC GAG GTA GCT CGC GCC ATC GAC GCC GCA TCG CGC GAC ACA
GGC TTC TTT TAC GCG GTG AAC CAC GGT GTC GAC CTG CCG TGG
CTC TCG CGC GAG ACG AAC AAA TTC CAC ATG AGC ATC ACG GAC
GAG GAG AAG TGG CAG CTC GCC ATC CGG GCC TAC AAC AAG GAG
CAC GAG TCC CAG ATC CGG GCG GGC TAC TAC CTG CCG ATC CCG
GGC AAG AAG GCG GTC GAA TCG TTC TGC TAC CTG AAC CCC TCC
TTC AGC CCA GAC CAC CCG CGA ATC AAG GAG CCC ACC CCT ATG
CAC GAG GTC AAC GTC TGG CCG GAC GAG GCG AAG CAC CCG GGG
TTC CGG GCC TTC GCC GAG AAG TAC TAC TGG GAC GTC TTC GGC
CTC TCC TCC GCG GTG CTG CGC GGC TAC GCT CTC GCC CTA GGT
CGC GAC GAG GAC TTC TTC ACC CGC CAC TCC CGC CGT GAC ACG
ACG CTC TCG TCG GTC GTG CTC ATC CGT TAC CCG TAC CTC GAC
CCG TAC CCG GAG CCG GCC ATC AAG ACG GCC GAC GAC GGC ACC
AAG CTC AGC TTC GAG TGG CAC GAG GAC GTG TCC CTC ATC ACG
GTG TTG TAC CAG TCC GAC GTG CAG AAT CTG CAG GTC AAG ACC
CCG CAG GGC TGG CAG GAC ATC CAG GCT GAC GAC ACG GGC TTC
CTC ATC AAC TGC GGC AGC TAC ATG GCC CAT ATC ACC GAC GAC
TAC TAC CCG GCC CCG ATC CAC CGC GTC AAA TGG GTC AAC GAG
GAG CGC CAG TCA CTG CCC TTC TTC GTC AAC CTG GGC TGG GAG
GAC ACC ATC CAG CCG TGG GAC CCC GCG ACC GCC AAG GAT GGG

GCC AAG GAT GCC GCC AAG GAC AAG CCG GCC ATC TCC TAC GGA
GAG TAT CTG CAG GGG GGA CTG CGG GGC TTG ATC AAC AAG AAT
GGT CAG ACC TAA-3'
```

wherein A is deoxyadenyl, G is deoxyguanyl, C is deoxycytidyl, and T is thymidyl.

4. A recombinant DNA vector which comprises a DNA compound of Claim 1, 2 or 3.

5. The vector of Claim 4 that is a plasmid.

6. The vector of Claim 5 which is plasmid pIT335(NRRL B-15960).

7. The vector of Claim 5 which is plasmid pIT337 constructed by ligating the ~1.6 kb NCOI-BamHI and ~8.7 kb Ncol-Ncol restriction fragments of plasmid pCZ106 (NRRL B 15959) to the ~1.5 kb Ncol-BamHI restriction fragment of plasmid pIT335 (NRRL B-15960).

8. The vector of Claim 4 which comprises the transcriptional and translational activating sequence of the Saccharomyces cerevisiae phosphoglycerate kinase gene positioned for expression of a DNA sequence that encodes a hygromycin resistance-conferring gene.

9. The vector of Claim 8 which is plasmid pPS20 constructed by inserting the ~2.7 kb HindIII restriction fragment of plasmid pIT221 comprising the transcriptional and translational activating sequence of the yeast Saccharomyces cerevisiae, into the single Hind III restriction enzyme recognition site of plasmid pIT335 as defined in claim 2, and represented in Figure 5; and its functionally equivalent isomer, plasmid pPS20.1.

10. A DNA compound which comprises the Cephalosporium acremonium transcriptional and translational activating sequence of the isopenicillin N synthetase gene as obtainable from plasmid pIT335(NRRL B-15960)

11. The compound of Claim 10 which is the ~0.5 kb SalI-NcoI restriction fragment of plasmid pIT335 obtainable from E. coli K12 JA221/pIT335 (NRRL B-15960).

12. The compound of Claim 10 or 11 which comprises the sequence:

```
5'-CGAATACTTG AATATTCCTT GGTCGCTCTT CTGATTTTCG
   |||||||||| |||||||||| |||||||||| ||||||||||
3'-GCTTATGAAC TTATAAGGAA CCAGCGAGAA GACTAAAAGC

   AGGCTTCTCC TTCCGCCATC GTCGCCTCAC GCATATCTCG
   |||||||||| |||||||||| |||||||||| ||||||||||
   TCCGAAGAGG AAGGCGGTAG CAGCGGAGTG CGTATAGAGC

   TCTTTCACAT CTTACACCAG CAGGACAAAC CGTCACC-3'
   |||||||||| |||||||||| |||||||||| |||||||
   AGAAAGTGTA GAATGTGGTC GTCCTGTTTG GCAGTGG-5'
```

wherein, A is deoxyadenyl, G is deoxyguanyl, C is deoxycytidyl, and T is thymidyl.

13. A recombinant DNA vector which comprises the DNA compound of Claim 10, 11 or 12.

14. The vector of Claim 13 which is a plasmid.

15. The vector of Claim 14 which is plasmid pPS23 constructed by isolating the ~850 bp NcoI restriction fragment of plasmid pIT335 that comprises the activating sequence of the isopenicillin N synthetase gene, attaching linkers with BamHI and NcoI-compatible, single-stranded overlaps to the ~850 bp NcoI fragment, and ligating the resulting plasmid pIT335-derived, ~860 bp BamHI restriction fragment to BamHI-digested plasmid pUC8; and its isomer plasmid pPS23.1.

16. The vector of Claim 14 in which the Cephalosporium acremonium transcriptional and translational activating sequence is positioned for expression of a DNA sequence that encodes a functional polypeptide.

17. The vector of Claim 16 wherein the functional polypeptide is an antibiotic biosynthetic enzyme.

18. The vector of Claim 16 wherein the functional polypeptide is an antibiotic resistance-conferring enzyme.

19. The vector of Claim 18 wherein the antibiotic resistance-conferring enzyme confers resistance to hygromycin.

20. The vector of Claim 17, 18 or 19 which is:

plasmid pPS21 derived by first removing the ~300 bp XmaI fragment of plasmid pIT221 to form plasmid pPS19 then digesting with BamHI and treating with the Klenow fragment of E. coli DNA Polymerase I and ligating the resulting ~7.7 Kb BamHI fragment to the ~0.8 kb, Klenow-treated, NcoI restriction fragment of plasmid pIT335;

EP 0 200 425 B1

plasmid pPS21A derived by ligating the ∿0.86 bp BamHI restriction fragment from digestion of plasmid pPS23 defined above with the ∿7.7 kb BamHI restriction fragment of plasmid pPS19 defined above;

plasmid pPS25 produced by ligating the two BamHI restriction fragments of plasmid pPS19 to the ∿860 bp BamHI restriction fragment of plasmid pPS23, as shown in Figure 9;

plasmid pPS25.1 having the same structure as pPS25 except with respect to the orientation of the ∿0.23 kb BamHI restriction fragment;

plasmid pP526 derived by inserting the ∿3.45 kb HindIII restriction fragment of plasmid pPS21A into the single HindIII site of plasmid pIT335;

plasmid pPS26.1 having the same structure as pPS26 except with respect to the orientation of the inserted HindIII restriction fragment;

plasmid pPS27 produced by inserting the ∿1.4 kb BamHI-XhoI restriction fragment of plasmid pIT335 into SalI-BamHI-digested plasmid pUC8 to give plasmid pIT336 which is digested with PstI and recircularized to give plasmid pPS35 which in turn is digested with HindIII and the ∿2.3 kb HindIII restriction fragment of pPS29 inserted;

plasmid pPS28 derived by deleting the ∿1.85 kb PstI restriction fragment from plasmid pPS21A;

plasmid pPS29 derived by deleting the ∿1.85 kb PstI and ∿0.49 kb PstI restriction fragments from plasmid pPS21A;

plasmid pPS30 constructed by inserting the ∿3.7 kb XmaI restriction fragment of plasmid pPS6 into the single XmaI site of plasmid pPS21A;

plasmid pPS30.1 having the same structure as pP230 except with respect to the orientation of the XmaI restriction fragment;

plasmid pPS31 constructed by inserting the ∿3.7 kb XmaI restriction fragment of plasmid pPS6 into the single XmaI site of plasmid pPS29;

plasmid pPS31.1 having the same structure as pPS31 except with respect to the orientation of the XmaI restriction fragment;

plasmid pPS37 constructed by inserting the ∿3.45 kb HindIII restriction fragment of plasmid pPS21A into NruI-digested plasmid pIT336; or

plasmid pPS37.1 having the same structure as pPS37 except with respect to the orientation of the HindIII restriction fragment.

21. A DNA compound which encodes the transcription termination and mRNA polyadenylation and processing signals of the Cephalosporium acremonium IPS gene as obtainable from plasmid pIT335-(NRRL B-15960)

22. The compound of Claim 21 which is the ∿0.5 kb PstI-BamHI restriction fragment of plasmid pIT335 obtainable from E. coli K12 JA221/pIT335 (NRRL B-15960).

23. A recombinant DNA vector which comprises the compound of Claim 21 or 22.

24. The vector of Claim 23 which is a plasmid.

25. The vector of Claim 24 which is:

34

plasmid pIT336 constructed by inserting the ∿1.4 kb BamHI-XhoI restriction fragment of plasmid pIT335 obtainable from E. coli K12 JA221/pIT335 (NRRL B-15960) into SalI-BamHI - digested plasmid pUC8;

plasmid pPS34 constructed by inserting the ∿2.3 kb HindIII and ∿0.5 kb HindIII-BamHI restriction fragments of plasmid pPS27 defined in claim 20 into the ∿5.9 kb BglII-HindIII restriction fragment of plasmid pIT335; or

plasmid pP235 constructed by digesting plasmid pIT336 prepared by inserting the ∿1.4 kb BamHI-XhoI restriction fragment of plasmid pIT335 into SalI-BamHI digested plasmid pUC8, with PstI and recircularizing to delete all of the Cephalosporium DNA sequences except the ∿0.5 kb BamHI-PstI restriction fragment.

26. A method of producing isopenicillin N synthetase activity in a host cell which comprises:
1) transforming said host cell with a recombinant DNA expression vector comprising the DNA compound of Claim 1, 2 or 3 positioned for expression from a transcriptional and translational activating sequence functional in said host cell; and
2) culturing said host cell transformed in step (1) under conditions that allow for expression of said DNA.

27. The method of Claim 26 in which the DNA compound is the ∿1.5 kb NcoI-BamHI restriction fragment plasmid pIT335 obtainable from E. coli K12 JA221/pIT335 (NRRL B-15960).

28. The method of Claim 26 or 27 in which the DNA compound encodes the amino acid sequence:

```
MET GLY SER VAL PRO VAL PRO VAL ALA ASN VAL PRO ARG ILE
ASP VAL SER PRO LEU PHE GLY ASP ASP LYS GLU LYS LYS LEU
GLU VAL ALA ARG ALA ILE ASP ALA ALA SER ARG ASP THR GLY
PHE PHE TYR ALA VAL ASN HIS GLY VAL ASP LEU PRO TRP LEU
SER ARG GLU THR ASN LYS PHE HIS MET SER ILE THR ASP GLU
GLU LYS TRP GLN LEU ALA ILE ARG ALA TYR ASN LYS GLU HIS
GLU SER GLN ILE ARG ALA GLY TYR TYR LEU PRO ILE PRO GLY
LYS LYS ALA VAL GLU SER PHE CYS TYR LEU ASN PRO SER PHE
SER PRO ASP HIS PRO ARG ILE LYS GLU PRO THR PRO MET HIS
GLU VAL ASN VAL TRP PRO ASP GLU ALA LYS HIS PRO GLY PHE
ARG ALA PHE ALA GLU LYS TYR TYR TRP ASP VAL PHE GLY LEU
SER SER ALA VAL LEU ARG GLY TYR ALA LEU ALA LEU GLY ARG
ASP GLU ASP PHE PHE THR ARG HIS SER ARG ARG ASP THR THR
LEU SER SER VAL VAL LEU ILE ARG TYR PRO TYR LEU ASP PRO
TYR PRO GLU PRO ALA ILE LYS THR ALA ASP ASP GLY THR LYS
LEU SER PHE GLU TRP HIS GLU ASP VAL SER LEU ILE THR VAL
LEU TYR GLN SER ASP VAL GLN ASN LEU GLN VAL LYS THR PRO
GLN GLY TRP GLN ASP ILE GLN ALA ASP ASP THR GLY PHE LEU
ILE ASN CYS GLY SER TYR MET ALA HIS ILE THR ASP ASP TYR
TYR PRO ALA PRO ILE HIS ARG VAL LYS TRP VAL ASN GLU GLU
ARG GLN SER LEU PRO PHE PHE VAL ASN LEU GLY TRP GLU ASP
THR ILE GLN PRO TRP ASP PRO ALA THR ALA LYS ASP GLY ALA
LYS ASP ALA ALA LYS ASP LYS PRO ALA ILE SER TYR GLY GLU
TYR LEU GLN GLY GLY LEU ARG GLY LEU ILE ASN LYS ASN GLY
GLN THR
```

wherein ALA is an alanine residue, ARG is an arginine residue, ASN is an asparagine residue, ASP is an aspartic acid residue, CYS is a cysteine residue, GLN is a glutamine residue, GLU is a glutamic acid residue, GLY is a glycine residue, HIS is a histidine residue, ILE is an isoleucine residue, LEU is a leucine residue, LYS is a lysine residue, MET is a methionine residue, PHE is a phenylalanine residue, PRO is a proline residue, SER is a serine residue, THR is a threonine residue, TRP is a tryptophan residue, TYR is a tyrosine residue, and VAL is a valine residue.

29. The method of Claim 26, 27 or 28 wherein the host cell is of the genus Agrobacterium, Cephalosporium, Chromobacterium, E. coli, Gluconobacter, Nocardia, Penicillium, Serratia, or Streptomyces.

30. The method of Claim 29 wherein the host cell is Cephalosporium acremonium, Penicillin chrysogenum or E. coli.

31. The method of any of Claims 26 to 30 in which the host cell cultured is E. coli/pIT337 obtained by transforming E. coli with plasmid pIT337 prepared by isolating pCZ106 from E. coli K12 RV308/pCZ106 (NRRL B-15959), digesting and isolating the ~8.7 kb NcoI-NcoI and ~1.6 kb NcoI-BamHI fragments, and ligating these fragments with the ~1.5 kb NcoI-BamHI restriction fragment of pIT335 obtainable from E. coli K12 JA221/pIT335 (NRRL B-15960).

32. The method of any of Claims 26 to 30 in which the host cell cultured is Cephalosporium acremonium/-pPS20 obtained by transforming Cephalosporium acremonium with plasmid pPS20 as defined in Claim 9.

33. A host cell transformed with the recombinant DNA vector of any of Claims 4 to 9 or 13 to 20.

34. The host cell of Claim 33 which is E. coli.

35. The host cell of Claim 33 which is Cephalosporium acremonium.

36. The host cell of Claim 34 which is E. coli K12/pIT335 (NRRL B-15960).

37. The host cell of Claim 35 which is Cephalosporium acremonium/pPS21 obtained by transforming Cephalosporium acremonium with plasmid pPS21 as defined in Claim 20.

**Revendications**

1. Composé d'ADN sélectionné à partir :
   (a) du fragment de restriction **NcoI-Bam**HI d'environ 1,5 kb du plasmide pIT335, que l'on peut obtenir à partir de **E. coli** K12 JA221/pIT335 (NRRL B-15960), et
   (b) des séquences d'ADN qui s'hybrident au composé d'ADN susmentionné, dans des conditions strictes, et qui codent pour une synthétase de l'isopénicilline N.

2. Composé d'ADN selon la revendication 1, à savoir le fragment de restriction **NcoI-Bam**HI d'environ 1,5 kb du plasmide pIT335.

3. Composé d'ADN selon la revendication 1, dans lequel la séquence du brin codant est :

```
5'-ATG GGT TCC GTT CCA GTT CCA GTG GCC AAC GTC CCC CGA
ATC GAT GTC TCG CCC CTA TTC GGC GAT GAC AAG GAG AAG AAG
CTC GAG GTA GCT CGC GCC ATC GAC GCC GCA TCG CGC GAC ACA
GGC TTC TTT TAC GCG GTG AAC CAC GGT GTC GAC CTG CCG TGG
CTC TCG CGC GAG ACG AAC AAA TTC CAC ATG AGC ATC ACG GAC
GAG GAG AAG TGG CAG CTC GCC ATC CGG GCC TAC AAC AAG GAG
CAC GAG TCC CAG ATC CGG GCG GGC TAC TAC CTG CCG ATC CCG
GGC AAG AAG GCG GTC GAA TCG TTC TGC TAC CTG AAC CCC TCC
TTC AGC CCA GAC CAC CCG CGA ATC AAG GAG CCC ACC CCT ATG
CAC GAG GTC AAC GTC TGG CCG GAC GAG GCG AAG CAC CCG GGG
TTC CGG GCC TTC GCC GAG AAG TAC TAC TGG GAC GTC TTC GGC
CTC TCC TCC GCG GTG CTG CGC GGC TAC GCT CTC GCC CTA GGT
CGC GAC GAG GAC TTC TTC ACC CGC CAC TCC CGC CGT GAC ACG
ACG CTC TCG TCG GTC GTG CTC ATC CGT TAC CCG TAC CTC GAC
CCG TAC CCG GAG CCG GCC ATC AAG ACG GCC GAC GAC GGC ACC
AAG CTC AGC TTC GAG TGG CAC GAG GAC GTG TCC CTC ATC ACG
GTG TTG TAC CAG TCC GAC GTG CAG AAT CTG CAG GTC AAG ACC
```

37

```
CCG CAG GGC TGG CAG GAC ATC CAG GCT GAC GAC ACG GGC TTC
CTC ATC AAC TGC GGC AGC TAC ATG GCC CAT ATC ACC GAC GAC
TAC TAC CCG GCC CCG ATC CAC CGC GTC AAA TGG GTC AAC GAG
GAG CGC CAG TCA CTG CCC TTC TTC GTC AAC CTG GGC TGG GAG
GAC ACC ATC CAG CCG TGG GAC CCC GCG ACC GCC AAG GAT GGG
```

**GCC AAG GAT GCC GCC AAG GAC AAG CCG GCC ATC TCC TAC GGA
GAG TAT CTG CAG GGG GGA CTG CGG GGC TTG ATC AAC AAG AAT
GGT CAG ACC TAA-3'**

dans laquelle A représente un groupe désoxyadényle, G représente un groupe désoxyguanyle, C représente un groupe désoxycytidyle et T représente un groupe thymidyle.

4. Vecteur d'ADN recombinant qui comprend un composé d'ADN selon la revendication 1, 2 ou 3.

5. Vecteur selon la revendication 4, qui est un plasmide.

6. Vecteur selon la revendication 5, à savoir le plasmide pIT335 (NRRL B-15960).

7. Vecteur selon la revendication 5, à savoir le plasmide pIT337 que l'on construit en ligáturant les fragments de restriction **NcoI-BamHI** d'environ 1,6 kb et **NcoI-NcoI** d'environ 8,7 kb du plasmide pCZ106 (NRRL B-15959), au fragment de restriction **NcoI-BamHI** d'environ 1,5 kb du plasmide pIT335 (NRRL B-15960).

8. Vecteur selon la revendication 4, qui comprend la séquence d'activation de transcription et de translation du gène de phosphoglycérate kinase de **Saccharomyces cerevisiae** positionné pour l'expression d'une séquence d'ADN qui encode un gène conférant de la résistance à l'hygromycine.

9. Vecteur selon la revendication 8, à savoir le plasmide pPS20 que l'on construit en insérant le fragment de restriction **HindIII** d'environ 2,7 kb du plasmide pIT221, comprenant la séquence d'activation de transcription et de translation de **Saccharomyces cerevisiae** de la levure, dans le site unique de reconnaissance d'enzyme de restriction **HindIII** du plasmide pIT335 tel que défini à la revendication 2 et représenté en figure 5; ainsi que son isomère à fonctionnalité équivalente, à savoir le plasmide pPS20.1.

10. Composé d'ADN qui comprend la séquence d'activation de transcription et de translation de **Cephalosporirn acremonium** du gène de synthétase de l'isopénicilline M, tel qu'on peut l'obtenir à partir du plasmide pIT335 (NRRL B-15960).

11. Composé selon la revendication 10, à savoir le fragment de restriction **SalI-NcoI** d'environ 0,5 kb du plasmide pIT335 que l'on peut obtenir à partir de **E. coli** K12 JA221/pIT335 (NRRL B-15960).

12. Composé selon la revendication 10 ou 11, qui comprend la séquence

```
5'-CGAATACTTG AATATTCCTT GGTCGCTCTT CTGATTTTCG
   |||||||||| |||||||||| |||||||||| ||||||||||
3'-GCTTATGAAC TTATAAGGAA CCAGCGAGAA GACTAAAAGC

  AGGCTTCTCC TTCCGCCATC GTCGCCTCAC GCATATCTCG
  |||||||||| |||||||||| |||||||||| ||||||||||
  TCCGAAGAGG AAGGCGGTAG CAGCGGAGTG CGTATAGAGC

  TCTTTCACAT CTTACACCAG CAGGACAAAC CGTCACC-3'
  |||||||||| |||||||||| |||||||||| |||||||
  AGAAAGTGTA GAATGTGGTC GTCCTGTTTG GCAGTGG-5'
```

dans laquelle A représente un groupe désoxyadényle, G représente un groupe désoxyguanyle, C représente un groupe désoxycytidyle et T représente un groupe thymidyle.

**13.** Vecteur d'ADN recombinant qui comprend le composé d'ADN selon la revendication 10, 11 ou 12.

**14.** Vecteur selon la revendication 13, qui est un plasmide.

**15.** Vecteur selon la revendication 14, à savoir le plasmide pPS23 que l'on construit en isolant le fragment de restriction **NcoI** d'environ 850 pb du plasmide pIT335, qui comprend la séquence d'activation du gène de synthétase de l'isopénicilline M, en fixant des chaînons munis de chevauchements monocaténaires compatibles de **BamHI** et de **NcoI** au fragment **NcoI** d'environ 850 pb et en ligaturant le fragment de restriction **BamHI** d'environ 860 pb résultant qui dérive du plasmide pIT335, au plasmide pUC8 mis à digérer par **BamHI**; ainsi que son plasmide isomère pPS23.1.

**16.** Vecteur selon la revendication 14, dans lequel la séquence d'activation de transcription et de translation de **cephalosporium acremonium** est positionnée pour l'expression d'une séquence d'ADN qui encode un polypeptide fonctionnel.

**17.** Vecteur selon la revendication 16, dans lequel le polypeptide fonctionnel est une enzyme biosynthétique d'antibiotique.

**18.** Vecteur selon la revendication 16, dans lequel le polypeptide fonctionnel est une enzyme conférant de la résistance à un antibiotique.

**19.** Vecteur selon la revendication 18, dans lequel l'enzyme conférant de la résistance à un antibiotique confère de la résistance à l'hygromycine.

**20.** Vecteur selon la revendication 17, 18 ou 19, à savoir :

le plasmide pPS21 que l'on dérive en retirant d'abord le fragment **XmaI** d'environ 300 pb du plasmide pIT221 pour obtenir le plasmide pPS19, puis en le mettant à digérer avec **BamHI** et en le traitant avec le fragment de Klenow de l'ADN polymérase I de **E. coli** et en ligaturant le fragment **BamHI** résultant d'environ 7,7 kb au fragment de restriction **NcoI** d'environ 0,8 kb, traité par Klenow, du plasmide pIT335;

le plasmide pPS21A que l'on dérive en ligaturant le fragment de restriction **BamHI** d'environ 0,86 pb provenant de la digestion du plasmide pPS23 tel que défini ci-dessus, avec le fragment de restriction **BamHI** d'environ 7,7 kb du plasmide pPS19 défini ci-dessus;
le plasmide pPS25 que l'on obtient en ligaturant les deux fragments de restriction **BamHI** du plasmide pPS19 au fragment de restriction **BamHI** d'environ 860 pb du plasmide pPS23, tel que représenté en figure 9;

le plasmide pPS25.1 qui possède la même structure que celle de pPS25, sauf en ce qui concerne l'orientation du fragment de restriction **BamHI** d'environ 0,23 kb;

le plasmide pPS26 que l'on dérive en insérant le fragment de restriction **Hin**dIII d'environ 3,45 kb du plasmide pPS21A dans le site unique **Hin**dIII du plasmide pIT335;

le plasmide pPS26.1 qui possède la même structure que celle de pPS26, sauf en ce qui concerne l'orientation du fragment de restriction inséré **Hin**dIII;

le plasmide pPS27 que l'on obtient en insérant le fragment de restriction **Bam**HI-**Xho**I d'environ 1,4 kb du plasmide pIT335 dans le plasmide pUC8 mis à digérer par **Sal**I-**Bam**HI, pour obtenir le plasmide pIT336 que l'on met à digérer par **Pst**I et que l'on remet en circulation pour obtenir le plasmide pPS35 que l'on met, à son tour, à digérer avec **Hin**dIII et le fragment de restriction **Hin**dIII d'environ 2,3 kb de pPS29 inséré;

le plasmide pPS28 que l'on dérive en supprimant le fragment de restriction **Pst**I d'environ 1,85 kb du plasmide pPS21A;

le plasmide pPS29 que l'on dérive en supprimant le fragment de restriction **Pst**I d'environ 1,85 kb et **Pst**I d'environ 0,49 kb du plasmide pPS21A;

le plasmide pPS30 que l'on construit en insérant le fragment de restriction **Xma**I d'environ 3,7 kb du plasmide pPS6 dans le site unique **Xma**I du plasmide pPS21A;

le plasmide pPS30.1 qui possède la même structure que celle de pPS30, sauf en ce qui concerne l'orientation du fragment de restriction **Xma**I;

le plasmide pPS31 que l'on construit en insérant le fragment de restriction **Xma**I d'environ 3,7 kb du plasmide pPS6 dans le site unique **Xma**I du plasmide pPS29;

le plasmide pPS31.1 qui possède la même structure que celle de pPS31, sauf en ce qui concerne l'orientation du fragment de restriction **Xma**I;

le plasmide pPS37 que l'on construit en insérant le fragment de restriction **Hin**dIII d'environ 3,45 kb du plasmide pPS21A dans le plasmide pIT336 mis à digérer par **Nru**I; ou

le plasmide pPS37.1 qui possède la même structure que celle de pPS37, sauf en ce qui concerne l'orientation du fragment de restriction **Hin**dIII.

21. Composé d'ADN qui encode la terminaison de transcription et les signaux de traitement et de polyadénylation de l'ARN messager du gène IPS de **Cephalosporium acremonium** tel qu'on peut l'obtenir à partir du plasmide pIT335 (NRRL B-15960).

22. Composé selon la revendication 21, à savoir le fragment de restriction **Pst**I-**Bam**HI d'environ 0,5 kb du plasmide pIT335, que l'on peut obtenir à partir de **E. coli** K12 JA221/pIT335 (NRRL B-15960).

23. Vecteur d'ADN recombinant qui comprend le composé selon la revendication 21 ou 22.

24. Vecteur selon la revendication 23, qui est un plasmide.

25. Vecteur selon la revendication 24, à savoir : le plasmide pIT336 que l'on construit en insérant le fragment de restriction **Bam**HI-**Xho**I d'environ 1,4 kb du plasmide pIT335 que l'on peut obtenir à partir de **E. coli** K12 JA221/pIT335 (NRRL B-15960) dans le plasmide pUC8 mis à digérer par **Sal**I-**Bam**HI;

le plasmide pPS34 que l'on construit en insérant les fragments de restriction **Hin**dIII d'environ 2,3 kb et **Hin**dIII-**Bam**HI d'environ 0,5 kb du plasmide pPS27 défini à la revendication 20, dans le fragment de restriction **Bgl**II-**Hin**dIII d'environ 5,9 kb du plasmide pIT335; ou

le plasmide pPS35 que l'on construit en mettant à digérer par **PstI** le plasmide pIT336 préparé par insertion du fragment de restriction **BamHI-XhoI** d'environ 1,4 kb du plasmide pIT335 dans lé plasmide pUC8 mis à digérer par **SalI-BamHI**, et en le remettant en circulation pour supprimer toutes les séquences d'ADN de **Cephalosporium,** à l'exception du fragment de restriction **BamHI-PstI** d'environ 0,5 kb.

**26.** Procédé pour obtenir l'activité de synthétase de l'isopénicilline N dans une cellule hôte, ce procédé consistant à :

1) transformer ladite cellule hôte avec un vecteur d'expression d'ADN recombinant comprenant le composé d'ADN selon la revendication 1, 2 ou 3, positionné pour l'expression à partir d'une séquence d'activation de transcription et de translation fonctionnelle dans ladite cellule hôte; et
2) cultiver ladite cellule hôte transformée à l'étape (1) dans des conditions qui permettent l'expression dudit ADN.

**27.** Procédé selon la revendication 26, dans lequel le composé d'ADN est constitué du fragment de restriction **NcoI-BamHI** d'environ 1,5 kb du plasmide pIT335 que l'on peut obtenir à partir de **E. coli** K12 JA221/pIT335 (NRRL B-1960).

**28.** procédé selon la revendication 26 ou 27, dans lequel le composé d'ADN encode la séquence d'acides aminés

```
MET GLY SER VAL PRO VAL PRO VAL ALA ASN VAL PRO ARG ILE
ASP VAL SER PRO LEU PHE GLY ASP ASP LYS GLU LYS LYS LEU
GLU VAL ALA ARG ALA ILE ASP ALA ALA SER ARG ASP THR GLY
PHE PHE TYR ALA VAL ASN HIS GLY VAL ASP LEU PRO TRP LEU
SER ARG GLU THR ASN LYS PHE HIS MET SER ILE THR ASP GLU
GLU LYS TRP GLN LEU ALA ILE ARG ALA TYR ASN LYS GLU HIS
GLU SER GLN ILE ARG ALA GLY TYR TYR LEU PRO ILE PRO GLY
LYS LYS ALA VAL GLU SER PHE CYS TYR LEU ASN PRO SER PHE
SER PRO ASP HIS PRO ARG ILE LYS GLU PRO THR PRO MET HIS
GLU VAL ASN VAL TRP PRO ASP GLU ALA LYS HIS PRO GLY PHE
ARG ALA PHE ALA GLU LYS TYR TYR TRP ASP VAL PHE GLY LEU
SER SER ALA VAL LEU ARG GLY TYR ALA LEU ALA LEU GLY ARG
ASP GLU ASP PHE PHE THR ARG HIS SER ARG ARG ASP THR THR
LEU SER SER VAL VAL LEU ILE ARG TYR PRO TYR LEU ASP PRO
TYR PRO GLU PRO ALA ILE LYS THR ALA ASP ASP GLY THR LYS
LEU SER PHE GLU TRP HIS GLU ASP VAL SER LEU ILE THR VAL
LEU TYR GLN SER ASP VAL GLN ASN LEU GLN VAL LYS THR PRO
GLN GLY TRP GLN ASP ILE GLN ALA ASP ASP THR GLY PHE LEU
ILE ASN CYS GLY SER TYR MET ALA HIS ILE THR ASP ASP TYR
TYR PRO ALA PRO ILE HIS ARG VAL LYS TRP VAL ASN GLU GLU
ARG GLN SER LEU PRO PHE PHE VAL ASN LEU GLY TRP GLU ASP
THR ILE GLN PRO TRP ASP PRO ALA THR ALA LYS ASP GLY ALA
LYS ASP ALA ALA LYS ASP LYS PRO ALA ILE SER TYR GLY GLU
TYR LEU GLN GLY GLY LEU ARG GLY LEU ILE ASN LYS ASN GLY
GLN THR
```

dans laquelle ALA représente un résidu d'alanine, ARG représente un résidu d'arginine, ASN représente un résidu d'asparagine, ASP représente un résidu d'acide aspartique, CYS représente un résidu de cystéine, GLN représente un résidu de glutamine, GLU représente un résidu d'acide glutamique, GLY représente un résidu de glycine, HIS représente un résidu d'histidine, ILE représente un résidu d'isoleucine, LEU représente un résidu de leucine, LYS représente un résidu de lysine, MET représente un résidu de méthionine, PHE représente un résidu de phénylalanine, PRO représente un résidu de proline, SER représente un résidu de sérine, THR représente un résidu de thréonine, TRP représente un résidu de tryptophane, TYR représente un résidu de tyrosine et VAL représente un résidu de valine.

29. Procédé selon la revendication 26, 27 ou 28, dans lequel la cellule hôte est du genre **Agrobacterium, cephalosporium, Chromobacterium, E. coli, Gluconobactar, Nocardia, Penicillium, Serratia** ou **Streptomyces.**

30. Procédé selon la revendication 29, dans lequel la cellule hôte est **cephalosporium acremonium, Penicillium chrysogenum** ou **E. coli.**

31. Procédé selon l'une quelconque des revendications 26 à 30, dans lequel la cellule hôte cultivée est **E. coli** K12/pIT337 que l'on obtient en transformant **E. coli** avec le plasmide pIT337 préparé en isolant pCZ106 à partir de **E. coli** K12 RV308/pCZ106 (NRRL B-15959), en mettant à digérer et en isolant les fragments **Ncol-Ncol** d'environ 8,7 kb et **Ncol-Bam**HI d'environ 1,6 kb et en ligaturant ces fragments au fragment de restriction **Ncol-Bam**HI d'environ 1,5 kb de pIT335 que l'on peut obtenir à partir de **E. coli** K12 JA221/pIT335 (NRRL B-15960).

32. Procédé selon l'une quelconque des revendications 26 à 30, dans lequel la cellule hôte cultivée est **Cephalosporium acremonium**/pPS20 que l'on obtient en transformant **cephalosporium acremonium** avec le plasmide pPS20 tel que défini à la revendication 9.

33. Cellule hôte transformée avec le vecteur d'ADN recombinant selon l'une quelconque des revendications 4 à 9 ou 13 à 20.

34. Cellule hôte selon la revendication 33, à savoir **E. coli.**

35. Cellule hôte selon la revendication 33, à savoir **Cephalosporium acremonium.**

36. Cellule hôte selon la revendication 34, à savoir **E. coli** K12/pIT335 (NRRL B-15960).

37. Cellule hôte selon la revendication 35, à savoir **Cephalosporium acremonium**/pPS21 que l'on obtient en transformant **Cephalosporium acremonium** avec le plasmide pPS21 tel que défini à la revendication 20.

## Patentansprüche

1. DNA-Verbindung, ausgewählt aus
   (a) dem ~1,5 kb Ncol-BamHI-Restriktionsfragment des Plasmids pIT335, erhältlich aus E. coli K12 JA221/pIT335 (NRRL B-15960) und
   (b) DNA-Sequenzen, die mit der vorhergehenden DNA-Verbindung unter stringenten Bedingungen hybridisieren und für eine Isopenicillin-N-Synthetase kodieren.

2. DNA-Verbindung nach Anspruch 1, die das ~1,5 kb Ncol-BamHI-Restriktionsfragment des Plasmids pIT335 ist.

3. DNA-Verbindung nach Anspruch 1, worin die Sequenz des kodierenden Stranges

```
5'-ATG GGT TCC GTT CCA GTT CCA GTG GCC AAC GTC CCC CGA
ATC GAT GTC TCG CCC CTA TTC GGC GAT GAC AAG GAG AAG AAG
CTC GAG GTA GCT CGC GCC ATC GAC GCC GCA TCG CGC GAC ACA
GGC TTC TTT TAC GCG GTG AAC CAC GGT GTC GAC CTG CCG TGG
CTC TCG CGC GAG ACG AAC AAA TTC CAC ATG AGC ATC ACG GAC
GAG GAG AAG TGG CAG CTC GCC ATC CGG GCC TAC AAC AAG GAG
CAC GAG TCC CAG ATC CGG GCG GGC TAC TAC CTG CCG ATC CCG
GGC AAG AAG GCG GTC GAA TCG TTC TGC TAC CTG AAC CCC TCC
TTC AGC CCA GAC CAC CCG CGA ATC AAG GAG CCC ACC CCT ATG
CAC GAG GTC AAC GTC TGG CCG GAC GAG GCG AAG CAC CCG GGG
TTC CGG GCC TTC GCC GAG AAG TAC TAC TGG GAC GTC TTC GGC
CTC TCC TCC GCG GTG CTG CGC GGC TAC GCT CTC GCC CTA GGT
CGC GAC GAG GAC TTC TTC ACC CGC CAC TCC CGC CGT GAC ACG
ACG CTC TCG TCG GTC GTG CTC ATC CGT TAC CCG TAC CTC GAC
CCG TAC CCG GAG CCG GCC ATC AAG ACG GCC GAC GAC GGC ACC
AAG CTC AGC TTC GAG TGG CAC GAG GAC GTG TCC CTC ATC ACG
GTG TTG TAC CAG TCC GAC GTG CAG AAT CTG CAG GTC AAG ACC
CCG CAG GGC TGG CAG GAC ATC CAG GCT GAC GAC ACG GGC TTC
CTC ATC AAC TGC GGC AGC TAC ATG GCC CAT ATC ACC GAC GAC
TAC TAC CCG GCC CCG ATC CAC CGC GTC AAA TGG GTC AAC GAG
GAG CGC CAG TCA CTG CCC TTC TTC GTC AAC CTG GGC TGG GAG
GAC ACC ATC CAG CCG TGG GAC CCC GCG ACC GCC AAG GAT GGG

GCC AAG GAT GCC GCC AAG GAC AAG CCG GCC ATC TCC TAC GGA
GAG TAT CTG CAG GGG GGA CTG CGG GGC TTG ATC AAC AAG AAT
GGT CAG ACC TAA-3'
```

ist, worin A Deoxyadenyl, G Deoxyguanyl, C Deoxycytidyl und T Thymidyl ist.

4. Rekombinanter DNA-Vektor, umfassend eine DNA-Verbindung nach Anspruch 1, 2 oder 3.

5. Vektor nach Anspruch 4, der ein Plasmid ist.

6. Vektor nach Anspruch 5, der das Plasmid pIT335 (NRRL B-15960) ist.

7. Vektor nach Anspruch 5, der das Plasmid pIT337 ist, der konstruiert wurde, indem das ∿1,6 kb NcoI-BamHI-Restriktionsfragment und das ∿8,7 kb NcoI-NcoI-Restriktionsfragment des Plasmids pC2106 (NRRL B-15959) mit dem ∿1,5 kb NcoI-BamHI-Restriktionsfragment des Plasmids pIT335 (NRRL B-15960) ligiert wird.

8. Vektor nach Anspruch 4, umfassend die transkriptions- und translationsaktivierende Sequenz des Phosphoglyceratkinasegens von Saccharomyces cerevisiae, das zur Expression einer DNA-Sequenz, die für ein hygromycinresistenzverleihendes Gen kodiert, in der Lage ist.

9. Vektor nach Anspruch 8, der das Plasmid pPS20 ist, das konstruiert wird, indem das ∿2,7 kb HindIII-Restriktionsfragment des Plasmids pIT221, das die transkriptions- und translationsaktivierende Sequenz der Hefe Saccharomyces cerevisiae enthält, in die einzige HindIII-Restriktionsenzymerkennungsstelle des Plasmids pIT335, wie in Anspruch 2 definiert und in Figur 5 dargestellt, insertiert wird und sein funktionell äquivalentes Isomer, Plasmid pPS20.1.

10. DNA-Verbindung, umfassend die transkriptions- und translationsaktivierende Sequenz des Isopenicillin-N-Synthetasegens von Cephalosporium acremonium, erhältlich aus Plasmid pIT335 (NRRL B-15960).

11. Verbindung nach Anspruch 10, die das ∿0,5 kb SalI-NcoI-Restriktionsfragment des Plasmids pIT335, erhältlich aus E. coli K12 JA221/pIT335 (NRRL B-15960) ist.

12. Verbindung nach Anspruch 10 oder 11, die die Sequenz

```
5'-CGAATACTTG AATATTCCTT GGTCGCTCTT CTGATTTTCG
    |||||||||| |||||||||| |||||||||| ||||||||||
3'-GCTTATGAAC TTATAAGGAA CCAGCGAGAA GACTAAAAGC

   AGGCTTCTCC TTCCGCCATC GTCGCCTCAC GCATATCTCG
   |||||||||| |||||||||| |||||||||| ||||||||||
   TCCGAAGAGG AAGGCGGTAG CAGCGGAGTG CGTATAGAGC

   TCTTTCACAT CTTACACCAG CAGGACAAAC CGTCACC-3'
   |||||||||| |||||||||| |||||||||| |||||||
   AGAAAGTGTA GAATGTGGTC GTCCTGTTTG GCAGTGG-5'
```

umfaßt, worin A Deoxyadenyl, G Deoxyguanyl, C Deoxycytidyl und T Thymidyl ist.

13. Rekombinanter DNA-Vektor, der die DNA-Verbindung von Anspruch 10, 11 oder 12 umfaßt.

14. Vektor nach Anspruch 13, der ein Plasmid ist.

15. Vektor nach Anspruch 14, der das Plasmid pPS23 ist, das konstruiert wurde, indem man das ∿850 bp NcoI-Restriktionsfragment des Plasmids pIT335, das die aktivierende Sequenz des Isopenicillin-N-Synthetasegens enthält, isoliert, an das ∿850 bp NcoI-Fragment Linker mit BamHI- und NcoI-kompatiblen, einzelsträngigen Überlappungen bindet und das entstehende, sich von dem Plasmid pIT335 ableitende ∿860 bp BamHI-Restriktionsfragment mit dem BamHI-verdauten Plasmid pUC8 ligiert, und dessen isomeres plasmid pPS23.1.

16. Vektor nach Anspruch 14, worin die transkriptions- und translationsaktivierende Sequenz von Cephalosporium acremonium für die Expression einer DNA-Sequenz, die für ein funktionelles Polypeptid kodiert, angeordnet ist.

17. Vektor nach Anspruch 16, worin das funktionelle Polypeptid ein antibiotisches biosynthetisches Enzym ist.

18. Vektor nach Anspruch 16, worin das funktionelle Polypeptid ein eine Antibiotikaresistenz verleihendes Enzym ist.

19. Vektor nach Anspruch 18, worin das die Antibiotikaresistenz verleihende Enzym eine Resistenz gegenüber Hygromycin verleiht.

EP 0 200 425 B1

**20.** Vektor nach Anspruch 17, 18 oder 19, der

Plasmid pPS21, abgeleitet, indem zuerst das ∿300 bp XmaI-Fragment von Plasmid pIT221 entfernt wird, um Plasmid pPS19 zu bilden, das dann mit BamHI verdaut wird und mit dem Klenow-Fragment der E. coli DNA-Polymerase I behandelt wird und das entstehende ∿7,7 kb BamHI-Fragment mit dem ∿0,8 kb, Klenow-behandelten, NcoI-Restriktionsfragment von Plasmid pIT335 ligiert wird;

Plasmid pPS21A, abgeleitet, indem das ∿0,86 kb BamHI-Restriktionsfragment aus dem Verdau von Plasmid pPS23, wie oben definiert, mit dem ∿7,7 kb BamHI-Restriktionsfragment von dem oben definierten Plasmid pPS19 ligiert wird;

Plasmid pPS25, hergestellt, indem die zwei BamHI-Restriktionsfragmente von plasmid pPS19 mit dem ∿860 bp BamHI-Restriktionsfragment von plasmid pPS23, wie in Figur 9 gezeigt, ligiert werden;

Plasmid pPS25.1, das dieselbe Struktur wie pPS 25 hat, mit Ausnahme der Orientierung des ∿0,23 kb BamHI-Restriktions-fragments;

Plasmid pPS26, abgeleitet, indem das ∿3,45 kb HindIII-Restriktionsfragment von Plasmid pPS21A in die einzige HindIII-Stelle von Plasmid pIT335 insertiert wird;

Plasmid pPS26.1, das dieselbe Struktur wie pPS26 hat, mit Ausnahme der Orientierung des insertierten HindIII-Restriktionsfragments;

Plasmid pPS27, hergestellt, indem das ∿1,4 kb BamHI-XhoI-Restriktionsfragment von Plasmid pIT335 in das SalI-BamHI-verdaute Plasmid pUC8 insertiert wird, was Plasmid pIT336 ergibt, das mit PstI verdaut wird und rezirkularisiert wird, was Plasmid pPS35 ergibt, das wiederum mit HindIII verdaut wird, und das ∿2,3 kb HindIII-Restriktionsfragment von pPS29 insertiert wird;

Plasmid pPS28, das sich ergibt, indem das ∿1,85 kb PstI-Restriktionsfragment aus Plasmid pPS21A deletiert wird;

Plasmid pPS29, das sich ergibt, indem die ∿1,85 kb PstI- und 0,49 kb PstI-Restriktionsfragmente aus Plasmid pPS21A deletiert werden;

Plasmid pPS30, das konstruiert wird, indem das ∿3,7 kb XmaI-Restriktionsfragment von Plasmid pPS6 in die einzige XmaI-Stelle von Plasmid pPS21A insertiert wird;

Plasmid pPS30.1, das dieselbe Struktur wie pPS30 hat mit Ausnahme der Orientierung des XmaI-Restriktionsfragmentes;

Plasmid pPS31, das konstruiert wird, indem das ∿3,7 kb XmaI-Restriktionsfragment von Plasmid pPS6 in die einzige XmaI-Stelle von Plasmid pPS29 insertiert wird;

Plasmid pPS31.1, das dieselbe Struktur wie pPS31 hat mit Ausnahme der Orientierung des XmaI-Restriktionsfragmentes;

Plasmid pPS37, das konstruiert wird, indem das ∿3,45 kb HindIII-Restriktionsfragment von Plasmid pPS21A in das mit NruI verdaute Plasmid pIT336 insertiert wird; oder

Plasmid pPS37.1 ist, das dieselbe Struktur wie pPS37 hat mit Ausnahme der Orientierung des HindIII-Restriktionsfragmentes.

**21.** DNA-Verbindung, die die Transkriptionstermination und mRNA-Polyadenylierung und Prozessierungssignale des IPS Gens von Cephalosporium acremonium kodiert, erhältlich aus Plasmid pIT335 (NRRL B-15960).

**22.** Verbindung nach Anspruch 21, die das 0,5 kb PstI-BamHI-Restriktionsfragment von Plasmid pIT335 ist, erhältlich aus E. coli K12 JA221/pIT335 (NRRL B-15960).

46

23. Rekombinanter DNA-Vektor, umfassend die Verbindung von Anspruch 21 oder 22.

24. Vektor nach Anspruch 23, der ein Plasmid ist.

25. Vektor nach Anspruch 24, der Plasmid pIT336, das konstruiert wird, indem das ∿1,4 kb BamHI-XhoI-Restriktionsfragment von Plasmid pIT335, erhältlich aus E. coli K12 JA221/pIT335 (NRRL B-15960) in ein mit SalI-BamHI verdautes Plasmid pUC8 insertiert wird;

Plasmid pPS34, das konstruiert wird, indem die ∿2,3 kb HindIII- und ∿0,5 kb HindIII-BamHI-Restriktionsfragmente von Plasmid pPS27, wie in Anspruch 20 definiert, in das ∿5,9 kb BglII-HindIII-Restriktionsfragment von Plasmid pIT335 insertiert werden; oder

Plasmid pPS35 ist, das konstruiert wird, indem Plasmid pIT336, das hergestellt wird, indem das ∿1,4 kb BamHI-XhoI-Restriktionsfragment von Plasmid pIT335 in ein mit SalI-BamHI verdautes Plasmid pUC8 insertiert wird, mit PstI verdaut wird und rezirkularisiert wird, um alle Sequenzen der Cephalosporium-DNA mit Ausnahme des ∿0,5 kb BamHI-PstI-Restriktionsfragmentes zu deletieren.

26. Verfahren zur Herstellung von Isopenicillin-N-Synthetaseaktivität in einer Wirtszelle, umfassend, daß man

1) die Wirtszelle mit einem rekombinanten DNA-Expressionsvektor, der die DNA-Verbindung von Anspruch 1, 2 oder 3 umfaßt, die so angeordnet ist, daß sie von der transkriptions- und translationsaktivierenden Sequenz, die in der Wirtszelle funktionell ist, exprimiert wird, transformiert und
2) die in Stufe 1) transformierte Wirtszelle unter solchen Bedingungen züchtet, die die Expression der DNA zulassen.

27. Verfahren nach Anspruch 26, worin die DNA-Verbindung das 1,5 kb NcoI-BamHI-Restriktionsfragment von Plasmid pIT335, erhältlich aus E. coli K12 JA221/pIT335 (NRRL B-15960) ist.

28. Verfahren nach Anspruch 26 oder 27, worin die DNA-Verbindung die folgende Aminosäuresequenz kodiert:

```
MET GLY SER VAL PRO VAL PRO VAL ALA ASN VAL PRO ARG ILE
ASP VAL SER PRO LEU PHE GLY ASP ASP LYS GLU LYS LYS LEU
GLU VAL ALA ARG ALA ILE ASP ALA ALA SER ARG ASP THR GLY
PHE PHE TYR ALA VAL ASN HIS GLY VAL ASP LEU PRO TRP LEU
SER ARG GLU THR ASN LYS PHE HIS MET SER ILE THR ASP GLU
GLU LYS TRP GLN LEU ALA ILE ARG ALA TYR ASN LYS GLU HIS
GLU SER GLN ILE ARG ALA GLY TYR TYR LEU PRO ILE PRO GLY
LYS LYS ALA VAL GLU SER PHE CYS TYR LEU ASN PRO SER PHE
SER PRO ASP HIS PRO ARG ILE LYS GLU PRO THR PRO MET HIS
```

```
GLU VAL ASN VAL TRP PRO ASP GLU ALA LYS HIS PRO GLY PHE
ARG ALA PHE ALA GLU LYS TYR TYR TRP ASP VAL PHE GLY LEU
SER SER ALA VAL LEU ARG GLY TYR ALA LEU ALA LEU GLY ARG
ASP GLU ASP PHE PHE THR ARG HIS SER ARG ARG ASP THR THR
LEU SER SER VAL VAL LEU ILE ARG TYR PRO TYR LEU ASP PRO
TYR PRO GLU PRO ALA ILE LYS THR ALA ASP ASP GLY THR LYS
LEU SER PHE GLU TRP HIS GLU ASP VAL SER LEU ILE THR VAL
LEU TYR GLN SER ASP VAL GLN ASN LEU GLN VAL LYS THR PRO
GLN GLY TRP GLN ASP ILE GLN ALA ASP ASP THR GLY PHE LEU
ILE ASN CYS GLY SER TYR MET ALA HIS ILE THR ASP ASP TYR
TYR PRO ALA PRO ILE HIS ARG VAL LYS TRP VAL ASN GLU GLU
ARG GLN SER LEU PRO PHE PHE VAL ASN LEU GLY TRP GLU ASP
THR ILE GLN PRO TRP ASP PRO ALA THR ALA LYS ASP GLY ALA
LYS ASP ALA ALA LYS ASP LYS PRO ALA ILE SER TYR GLY GLU
TYR LEU GLN GLY GLY LEU ARG GLY LEU ILE ASN LYS ASN GLY
GLN THR
```

worin ALA ein Alaninrest, ARG ein Argininrest, ASN ein Asparaginrest, ASP ein Asparginsäurerest, CYS ein Cysteinrest, GLN ein Glutaminrest, GLU ein Glutaminsäurerest, GLY ein Glycinrest, HIS ein Histidinrest, ILE ein Isoleucinrest, LEU ein Leucinrest, LYS ein Lysinrest, MET ein Methioninrest, PHE ein Phenylalaninrest, PRO ein Prolinrest, SER ein Serinrest, THR ein Threoninrest, TRP ein Tryptophanrest, TYR ein Tyrosinrest und VAL ein Valinrest ist.

29. Verfahren nach Anspruch 26, 27 oder 28, worin die Wirtszelle zur Gattung Agrobacterium, Cephalosporium, Chromobacterium, E. coli, Gluconobacter, Nocardia, Penicillium, Serratia oder Streptomyces gehört.

30. Verfahren nach Anspruch 29, worin die Wirtszelle Cephalosporium acremonium, Penicillium chrysogenum oder E. coli ist.

31. Verfahren nach einem der Ansprüche 26 bis 30, bei dem die gezüchtete Wirtszelle E. coli/pIT337 ist, erhältlich durch Transformieren von E. coli mit dem Plasmid pIT337, das hergestellt wird, indem pCZ106 aus E. coli K12 RV308/pCZ106 (NRRL B-15959) isoliert wird, verdaut wird und die ∼8,7 kb Ncol-Ncol- und ∼1,6 kb Ncol-BamHI-Fragmente isoliert werden und diese Fragmente mit dem ∼1,5 kb Ncol-BamHI-Restriktionsfragment aus pIT335, erhältlich aus E. coli K12 JA221/pIT335 (NRRL B-15960) ligiert werden.

32. Verfahren nach einem der Ansprüche 26 bis 30, wobei die gezüchtete Wirtszelle Cephalosporium acremonium/pPS20 ist, die erhalten wird, indem Cephalosporium acremonium mit Plasmid pPS20, wie in Anspruch 9 definiert, transformiert wird.

33. Wirtszelle, transformiert mit dem rekombinanten DNA-Vektor nach einem der Ansprüche 4 bis 9 oder 13 bis 20.

34. Wirtszelle nach Anspruch 33, die E. coli ist.

35. Wirtszelle nach Anspruch 33, die Cephalosporium acremonium ist.

36. Wirtszelle nach Anspruch 34, die E. coli K12/pIT335 (NRRL B-15960) ist.

37. Wirtszelle nach Anspruch 35, die Cephalosporium acremonium/pPS21 ist, die erhalten wird, indem Cephalosporium acremonium mit Plasmid pPS21, wie in Anspruch 20 definiert, transformiert wird.

# FIG.I

Restriction Site and Function Map of
Plasmid pIT335
(8.24 kb)

# FIG.2

Restriction Site and Function Map of
Plasmid pCZ106
(10.8 kb)

# FIG.3

Restriction Site and Function Map of
Plasmid pIT337
(11.8 kb)

# FIG. 4

Restriction Site and Function Map of
Plasmid pIT221
(8.04 kb)

# FIG. 5

Restriction Site and Function Map of
Plasmid pPS20
(11 kb)

# FIG.6

**Restriction Site and Function Map of
Plasmid pPS19
(7.85 kb)**

# FIG.7

Restriction Site and Function Map of
Plasmid pPS21
(8.5 kb)

# FIG.8

Restriction Site and Function Map of
Plasmid pPS21A
(8.5 kb)

# FIG.9

Restriction Site and Function Map of
Plasmid pPS25
(8.71 kb)

# FIG. IO

Restriction Site and Function Map of
Plasmid pPS28
(6.6 kb)

# FIG.11

Restriction Site and Function Map of
Plasmid pPS29
(6.1 kb)

# FIG.12

Restriction Site and Function Map of
Plasmid pPS26
(11.6 kb)

# FIG.13

Restriction Site and Function Map of
Plasmid pPS34
(8.7 kb)

# FIG.14

Restriction Site and Function Map of
Plasmid pIT336
(4.1 kb)

# FIG.15

Restriction Site and Function Map of
Plasmid pPS35
(4.1 kb)

# FIG.16

**Restriction Site and Function Map of Plasmid pPS27 (5.5 kb)**

# FIG.17

Restriction Site and Function Map of
Plasmid pPS37
(6.4 kb)